# EUROPÄISCHE PATENTSCHRIFT

(11) **EP 3 584 004 B1**
(45) Veröffentlichungstag und Bekanntmachung des Hinweises auf die Patenterteilung: **23.09.2020**
(21) Anmeldenummer: 19176769.8
(22) Anmeldetag: 27.05.2019
(51) Int. Cl.: B01F 11/00, B01F 13/00, B01F 15/00, B01F 15/02, A61B 17/88

(54) **KNOCHENZEMENTAPPLIKATOR MIT VERSENKBAREM MISCHSTAB UND VERFAHREN ZUR HERSTELLUNG EINES KNOCHENZEMENTS**
BONE CEMENT APPLICATOR WITH RETRACTABLE MIXING ROD AND METHOD FOR PRODUCING A BONE CEMENT
APPLICATEUR DE CIMENT OSSEUX POURVU DE BÂTON MÉLANGEUR POUVANT ÊTRE ABAISSÉ ET PROCÉDÉ DE FABRICATION D'UN CIMENT OSSEUX

(30) Priorität: 18.06.2018 DE 102018209784
(43) Veröffentlichungstag der Anmeldung: 25.12.2019
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- WO-A1-97/18031
- WO-A1-99/22854
- WO-A1-2011/089480
- DE-A1-102016 121 607
- US-A1- 2003 021 180

## Beschreibung

Die Erfindung betrifft einen Knochenzementapplikator zum Lagern und Mischen eines Knochenzementpulvers und einer Monomerflüssigkeit sowie zum Applizieren eines aus dem Knochenzementpulver und der Monomerflüssigkeit gemischten pastösen Knochenzements. Die Erfindung betrifft auch ein Verfahren zur Herstellung eines Knochenzements mit einem solchen Knochenzementapplikator.

Gegenstand der Erfindung ist ein Knochenzementapplikator zum Lagern, Mischen und Applizieren von Knochenzement. Die Knochenzementapplikator wird vorzugsweise durch ein geschlossenes Prepack-Mischsystem mit einer integrierten Auspressvorrichtung realisiert. Der Knochenzementapplikator ist vorzugsweise für die Arthoplasty, die Vertebroplasty und die Kyphoplasty geeignet beziehungsweise bestimmt. Es werden hierzu auch Verfahren zum Mischen und Applizieren von Polymethylmethacrylat-Knochenzement vorgeschlagen.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30). Konventionelle PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch Vermischen des Knochenzementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A. Diese Mischsysteme enthalten zur Vermischung der Zementkomponenten einen manuell von außen zu bedienenden Mischstab an dem Mischflügel als Mischer angebracht sind. Zur Erzeugung des Vakuums werden externe Vakuumpumpen benötigt. Diese Vakuumpumpen werden im Allgemeinen mit Druckluft angetrieben und erzeugen Vakuum nach dem Venturiprinzip. Für das Auspressen des gemischten Knochenzements aus den Kartuschen nutzt man manuell angetriebene Auspressvorrichtungen. Diese Auspressvorrichtungen können mit den Kartuschen zum Auspressen des Zementteigs reversibel verbunden werden. Nach dem Auspressvorgang werden die Auspressvorrichtungen von den Kartuschen getrennt, gereinigt und resterilisiert. Die verwendeten Kartuschen werden entsorgt.

Eine Weiterentwicklung in der Zementiertechnik stellen Zementiersysteme dar, in denen sowohl das Knochenzementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der WO 00/35506 A1, der EP 0 796 653 A2 und der US 5 588 745 A vorgeschlagen.

Das Patent DE 10 2009 031 178 B3 offenbart eine Lager- und Mischvorrichtung als Full-Prepacked-Mischvorrichtung, in dem die zur Herstellung des Knochenzements notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird. Für den Monomertransfer und das Mischen unter Vakuum wird eine externe Vakuumpumpe benötigt, die normalerweise durch Druckluft angetrieben wird. Zum Auspressen des gemischten Zementteigs wird ebenfalls eine separate, manuell zu bedienende Auspressvorrichtung benutzt.

In der DE 10 2016 121 607 A1 wird ein Full-Prepacked-Mischsystem mit einer Kartusche enthaltend ein Knochenzementpulver zur Herstellung eines Knochenzements vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Austragskolben, mit dem der Monomerflüssigkeitsbehälter zerquetscht werden kann und die Monomerflüssigkeit aus der Aufnahme in die Kartusche gepresst werden kann. Bei diesem System erfolgt keine manuelle Durchmischung der Ausgangskomponenten mit Hilfe eines Mischers.

In der WO 97/18031 A1 wird eine Vorrichtung zum sukzessiven Zuführen von Chargen in ein Mischgefäß unter Teilvakuum zur Herstellung von Knochenzement beschrieben.

In der WO 2011/089480 A1 wird eine Vorrichtung zum Herstellen und Verabreichen eines Knochenzements aus einer flüssigen Komponente und einer pulverförmigen Komponente beschrieben, wobei ein Mischstab durch eine Öffnung zum Austragen des gemischten Knochenzements geführt wird, um die beiden Komponenten zu mischen. In der WO 99/22854 A1 wird eine Vorrichtung zum Mischen mehrteiliger Reaktionsmaterialien unter Vakuum beschrieben, wobei die Vorrichtung ein Rührwerk zum Mischen des Reaktionsmaterials sowohl starre Schaufeln als auch flexible Paddel umfasst.

In der US 2003/021180 A1 wird eine Vorrichtung zum Mischen und Applizieren von Implantatmaterialien beschrieben, welche Mittel vorsieht, um eine Überlaufverbindung zwischen einem ersten Behälter und einem zweiten Behälter auf einem Mischmechanismus herzustellen, der sich auf einer Mischwelle befindet.

Bei den bisher referenzierten Vakuummischsystemen mit Mischer muss nach der Vermischung der Zementkomponenten, vor der Applikation des Knochenzements, der Mischstab entweder abgebrochen oder aus dem Mischsystem herausgezogen werden. Die bekannten Verfahren und Vorrichtungen haben dadurch den Nachteil, dass es beim Abbrechen zu einer Undichtigkeit des Knochenzementapplikators kommen kann und dass das Abbrechen sowie das Herausziehen des Mischstabs immer als zusätzliche Arbeitsschritte notwendig sind. Zudem liegt der abgebrochene Mischstab als ein weiteres separates Teil im OP-Saal herum, das entsorgt werden muss. Bei Knochenzementapplikatoren ohne Mischer muss einiger Aufwand betrieben werden, damit der Knochenzement ausreichend durchmischt ist. Zudem kann es stets dazu kommen, dass Teile des Knochenzements nicht ausreichend durchmischt sind. Diese müssen entfernt oder zurückgehalten werden oder es kann zu einer Beeinträchtigung der Qualität des Knochenzements kommen.

Die Erfindung hat die Aufgabe, einen Knochenzementapplikator zum Lagern, Mischen und Applizieren von Polymethylmethacrylat-Knochenzement zu entwickeln, mit dem die Nachteile des Stands der Technik überwunden werden. In diesem Knochenzementapplikator sollen das Knochenzementpulver und die Monomerflüssigkeit in separaten Kompartimenten vor ihrer Vermischung gelagert werden. Der Monomertransfer aus dem Monomerflüssigkeitsbehälter in das Knochenzementpulver soll ohne Anlegen eines extern bereitgestellten Vakuums erfolgen. Die Vermischung soll durch einen Mischstab mit einem Mischer in der geschlossenen Vorrichtung derart erfolgen, dass der medizinische Anwender nicht mit dem Knochenzementpulver oder der Monomerflüssigkeit in Berührung kommt. Nach der Vermischung der Zementkomponenten soll eine Entfernung des Mischstabs vom Mischsystem durch Herausziehen und/oder Abbrechen des Mischstabs vermieden werden. Der gebildete Knochenzement soll aus dem Knochenzementapplikator manuell auspressbar sein, ohne dass eine externe Auspressvorrichtung an die Vorrichtung angeschlossen werden muss.

Die Erfindung hat deshalb die Aufgabe, ein komplett autonomes Prepack-Mischystem zu entwickeln, das eine Vermischung der Zementkomponenten und ein Auspressen des gemischten Knochenzements erlaubt, ohne dass Zusatzgeräte wie externe Vakuumpumpen und Auspressvorrichtungen erforderlich sind.

Die Aufgaben der Erfindung werden gelöst durch einen Knochenzementapplikator zum Lagern und Mischen eines Knochenzementpulvers und einer Monomerflüssigkeit sowie zum Applizieren eines aus dem Knochenzementpulver und der Monomerflüssigkeit gemischten pastösen Knochenzements, der Knochenzementapplikator aufweisend
A) eine Kartusche mit einem zylindrischen Innenraum zum Mischen des Knochenzements, wobei die Kartusche an einer Vorderseite einen Kartuschenkopf mit einer Austragsöffnung zum Austreiben des Knochenzements aus dem Innenraum aufweist,
B) einen Austragskolben zum Austreiben des gemischten Knochenzements aus dem Innenraum durch die Austragsöffnung, der im Innenraum der Kartusche entlang der Zylinderachse des Innenraums in Richtung des Kartuschenkopfs beweglich angeordnet ist, wobei das Knochenzementpulver zwischen dem Austragskolben und dem Kartuschenkopf in dem Innenraum der Kartusche enthalten ist,
C) eine Aufnahme, wobei ein Monomerflüssigkeitsbehälter im Inneren der Aufnahme angeordnet ist, wobei der Monomerflüssigkeitsbehälter die Monomerflüssigkeit enthält und im Inneren der Aufnahme zu öffnen ist, wobei die Aufnahme an einer der Vorderseite der Kartusche gegenüberliegenden Rückseite der Kartusche in der Kartusche steckt und in der Kartusche beweglich ist, und
D) einen Mischstab, wobei der Mischstab mit einem daran befestigten Mischer in dem Innenraum der Kartusche angeordnet ist, wobei der Mischer an einer dem Kartuschenkopf zugewandten Vorderseite des Mischstabs befestigt ist, wobei der Mischstab an einer dem Mischer gegenüberliegenden Seite mit einer dem Kartuschenkopf zugewandten Vorderseite der Aufnahme verbunden ist, so dass der Mischstab mit dem Mischer in dem Innenraum zum Durchmischen des Knochenzementpulvers mit der Monomerflüssigkeit durch eine Bewegung der Aufnahme gegen die Kartusche beweglich ist, und wobei der Mischstab lösbar mit der Aufnahme verbunden ist und der von der Aufnahme gelöste Mischstab bei einem Vortreiben der Aufnahme in Richtung des Kartuschenkopfs in die Aufnahme hinein drückbar ist.

Bevorzugt ist die Aufnahme in der Kartusche zumindest axial in Richtung der Zylinderachse des Innenraums beweglich. Dabei ist die Aufnahme besonders bevorzugt zumindest bereichsweise in den Innenraum der Kartusche einschiebbar oder einschraubbar.

Die Aufnahme ist vorzugsweise ein Ampullenhalter.

Es kann vorgesehen sein, dass sich ein Teil der Aufnahme, insbesondere ein dem Kartuschenkopf zugewandter Verschluss der Aufnahme, mit dem Mischstab von der restlichen Aufnahme löst.

Bevorzugt ist der Monomerflüssigkeitsbehälter eine Ampulle aus Glas oder aus einem Kunststoff. Ampullen aus Glas oder Kunststoff sind besonders zuverlässig zu öffnen. Zudem kann die Monomerflüssigkeit in solchen Ampullen als Monomerflüssigkeitsbehälter besonders lange gelagert werden. Alternative Monomerflüssigkeitsbehälter könnten beispielsweise beschichtete Folienbeutel sein.

Die Aufnahme ist vorzugsweise als Ampullenhalter ausgebildet. Der Ampullenhalter ist dabei besonders bevorzugt zur Halterung einer Ampulle aus Glas oder Kunststoff geeignet und vorgesehen.

Der Innenraum der Kartusche hat eine zylindrische Geometrie mit kreisförmiger Grundfläche. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Der Innenraum der Kartusche benötigt aber eine rotationssymmetrische Symmetrie, das heißt eine zylindrische Form mit kreisförmiger Grundfläche, da ansonsten das Einschrauben der Aufnahme nicht möglich ist oder die Aufnahme an ihrer Vorderseite nicht gut gegen die Innenwand des Innenraums abgedichtet werden könnte.

Es kann bevorzugt vorgesehen sein, dass die Austragsöffnung für das Lagern und Mischen mit einem zu öffnenden Verschluss verschlossen ist. Hierdurch wird ein geschlossenes Prepack-Mischsystem bereitgestellt.

Dabei kann vorgesehen sein, dass der Verschluss über ein Gewinde oder einen Bajonett-Verschluss mit dem Kartuschenkopf lösbar verbunden ist.

Es kann vorgesehen sein, dass der Verschluss die Austragsöffnung flüssigkeitsdicht oder gasdicht und flüssigkeitsdicht verschließt.

Hiermit wird sichergestellt, dass beim Mischen des Knochenzements kein Knochenzementpulver, keine Monomerflüssigkeit und kein Knochenzement aus dem Innenraum der Kartusche austreten können.

Alle Teile des Knochenzementapplikators bis auf die Ausgangskomponenten, den Monomerflüssigkeitsbehälter und gegebenenfalls vorhandene Dichtungen bestehen erfindungsgemäß bevorzugt aus Kunststoff, insbesondere einem thermoplastischen Kunststoff. Der Monomerflüssigkeitsbehälter muss, wenn er aus einem Kunststoff besteht, aus einem spröden brechbaren Kunststoff bestehen. Die Dichtungen bestehen vorzugsweise aus Silikon oder Gummi.

Es kann vorgesehen sein, dass der Mischstab durch einen Druck auf den am Kartuschenkopf anliegenden Mischer und/oder durch ein Drehen oder Schrauben der Aufnahme gegen den gegen eine Drehung im Innenraum gesicherten Mischer von der Aufnahme lösbar ist.

Dadurch kann der Mischstab durch eine Bewegung der Aufnahme gegen den im Bereich des Kartuschenkopfs fixierten Mischer von der Aufnahme gelöst werden. Es bedarf dann keiner separaten Vorrichtung zum Lösen des Mischstabs vom Austragskolben. Der Knochenzementapplikator wird hierdurch im Aufbau vereinfacht.

Ferner kann vorgesehen sein, dass die Vorderseite der Aufnahme den Austragskolben zum Austreiben des Knochenzements aus dem Innenraum bildet, wobei bevorzugt der Austragskolben zylindrisch ist.

Dadurch muss der Mischstab nicht in Richtung der Zylinderachse des Innenraums beweglich durch einen separaten Austragskolben geführt sein. Dies hat allerdings den Nachteil, dass der Bereich des Innenraums, der an die Vorderseite des Austragskolbens grenzt, nicht von dem Mischer erreicht werden kann und also in diesem Bereich keine Durchmischung stattfindet. Wenn der Knochenzementapplikator allerdings mit dem Kartuschenkopf nach unten gehalten wird, sammelt sich das Knochenzementpulver und die auslaufende Monomerflüssigkeit aufgrund der Gravitation in dem vorderen Bereich des Innenraums, der an den Kartuschenkopf grenzt, und kann demzufolge von dem Mischer erreicht und durchmischt werden. Der Teil des Knochenzements, der am schlechtesten durchmischt wird, kann zudem in dem Knochenzementapplikator, beispielsweise in einem Totvolumen der Kartusche, zurückgehalten werden.

Alternative dazu kann vorgesehen sein, dass der Innenraum mit dem Austragskolben in einen vorderen Teil und einen hinteren Teil getrennt ist, wobei der vordere Teil des Innenraums durch den Kartuschenkopf und den Austragskolben begrenzt ist und der hintere Teil des Innenraums durch den Austragskolben und die Aufnahme begrenzt ist, wobei der Mischstab durch eine Durchführung in dem Austragskolben geführt ist und in der Durchführung axial beweglich gelagert ist, wobei der Mischer und das Knochenzementpulver im vorderen Teil des Innenraums angeordnet sind und wobei der Austragskolben mit der Aufnahme in Richtung des Kartuschenkopfs drückbar ist.

Hiermit kann sichergestellt werden, dass der gesamte vordere Teil des Innenraums, in dem der Knochenzement gemischt wird, von dem Mischer erreicht und damit durchmischt werden kann. Dazu kann der Mischer vorzugsweise bis zum Austragskolben gezogen werden. Dadurch sind alle Bereiche des vorderen Teils des Innenraums von dem Mischer erreichbar und es kann mit dem Knochenzementapplikator ein gut durchmischter Knochenzement erzeugt werden.

Bevorzugt weist der Austragskolben einen Kanal auf, der mit einer Porenscheibe abgedeckt ist oder in dem eine Porenscheibe angeordnet ist, oder der Austragskolben weist mehrere Kanäle auf, die mit einer Porenscheibe abgedeckt sind oder in denen jeweils eine Porenscheibe angeordnet ist. Der Kanal oder die Kanäle verbinden dann den vorderen Teil des Innenraums mit dem hinteren Teil des Innenraums, wobei die Verbindung für die Monomerflüssigkeit und Gase durchlässig ist aber aufgrund des Porenfilters oder anderer Maßnahmen für das Knochenzementpulver undurchlässig ist.

Wenn der Austragskolben als separates Teil in dem Innenraum der Kartusche vorgesehen ist, kann vorgesehen sein, dass der Austragskolben in Presspassung im Innenraum an der Kartusche fixiert ist, wobei vorzugsweise der Austragskolben einen zum Innenraum passenden zylindrischen oder kreisförmigen Außenumfang aufweist.

Hierdurch kann auf einfach Weise erreicht werden, dass einerseits der Mischstab durch den Austragskolben gleiten kann, ohne dass der Austragskolben gegen die Kartusche bewegt wird, aber andererseits der Austragskolben ohne zu großen Widerstand von der Aufnahme in Richtung des Kartuschenkopfs gedrückt werden kann, um den Knochenzement aus der Kartusche auszupressen und den Knochenzement durch den Austragskolben zu entgasen.

Ferner kann vorgesehen sein, dass der Austragskolben nicht durch eine Bewegung des Mischstabs in der Durchführung im Austragskolben innerhalb des Innenraums bewegbar ist.

Hiermit wird sichergestellt, dass der Mischer alle Bereiche des vorderen Teils des Innenraums der Kartusche erreichen kann und dadurch eine gute Durchmischung des Knochenzements erzielt werden kann.

Gemäß einer bevorzugten Weiterbildung der vorliegenden Erfindung kann vorgesehen sein, dass der Austragskolben zumindest einen für das Knochenzementpulver undurchlässigen und für die Monomerflüssigkeit und Gase durchlässigen Kanal hat.

Dadurch kann die Monomerflüssigkeit durch den Austragskolben hindurch zum Knochenzementpulver strömen, ohne dass das Knochenzementpulver vorher in Richtung des Monomerflüssigkeitsbehälters gelangen kann. Dadurch wird sichergestellt, dass der Knochenzement nur in dem Teil des Innenraums zwischen dem Austragskolben und dem Kartuschenkopf entsteht.

Des Weiteren kann vorgesehen sein, dass die Aufnahme bis zu einem durch ein Außengewinde an der Aufnahme gebildeten ersten Anschlag in den Innenraum hineinschiebbar ist und bis zu einem zweiten Anschlag mit dem Außengewinde in ein Innengewinde in der Kartusche an deren Rückseite oder in ein Innengewinde in einem Ring an der Rückseite der Kartusche einschraubbar ist, wobei der Mischstab bei einer Bewegung der Aufnahme bis zum ersten Anschlag nicht von der Aufnahme lösbar ist und der Mischstab durch Einschrauben der Aufnahme in die Kartusche von der Aufnahme lösbar ist.

Hiermit kann der Knochenzementapplikator auf einfache Weise bedient werden. Gleichzeitig wird so erreicht, dass sich der Mischstab nicht schon beim Mischen des Knochenzements von der Aufnahme löst, sondern erst beim Auspressen des gemischten Knochenzements, das durch Einschrauben der Aufnahme in die Kartusche, also in das Innengewinde an der Rückseite der Kartusche oder in das Innengewinde am Ring erfolgt.

Es kann auch vorgesehen sein, dass an der Aufnahme eine von außen bedienbare Öffnungseinrichtung angeordnet ist, mit der der Monomerflüssigkeitsbehälter im Inneren der Aufnahme zu öffnen ist.

Hierdurch kann der Monomerflüssigkeitsbehälter von außen aber gleichzeitig innerhalb der Aufnahme geöffnet werden. Dadurch kann verhindert werden, dass der Anwender mit der Monomerflüssigkeit in Kontakt kommt und dass Monomerflüssigkeit verloren geht, die zur Herstellung des Knochenzements vorgesehen ist und benötigt wird.

Dabei kann vorgesehen sein, dass der Monomerflüssigkeitsbehälter durch Einschieben oder Einschrauben der Öffnungseinrichtung in die Aufnahme zu öffnen ist, wobei sich der Monomerflüssigkeitsbehälter im Inneren der Aufnahme befindet.

Dadurch kann der gesamte Knochenzementapplikator durch ineinander schieben und/oder ineinander schrauben seiner Teile (der Öffnungseinrichtung in die Aufnahme und der Aufnahme in die Kartusche) bedient werden. Dadurch ist der Knochenzementapplikator besonders einfach anwendbar.

Dabei kann wiederum vorgesehen sein, dass der Monomerflüssigkeitsbehälter eine Ampulle aus Glas oder einem Kunststoff ist, wobei die Ampulle einen Ampullenkopf, einen zylindrischen Ampullenkörper und einen dem Ampullenkopf gegenüberliegenden Ampullenboden aufweist, wobei der Ampullenkopf einen kleineren Durchmesser als der Ampullenkörper hat und über Schultern mit dem Ampullenkörper verbunden ist, wobei die Öffnungseinrichtung eine Hülse aufweist, die beim Einschieben oder Einschrauben auf die Schultern der Ampulle drückt.

Die Hülse ist vorzugsweise durch einen Hohlzylinder realisiert, wobei eine Öffnung für einen Gasaustausch vorgesehen sein kann, um einen Überdruck in dem Knochenzementapplikator zu vermeiden.

Mit der Hülse kann ein gleichmäßiger Druck auf die Ampulle ausgeübt werden und so ein reproduzierbares Öffnen der Ampulle erreicht werden.

Dadurch, dass die Öffnungseinrichtung eine Hülse aufweist, die beim Einschieben oder Einschrauben auf die Schultern der Ampulle drückt, wird der Ampullenboden auf einen Vorsprung im Inneren der Aufnahme gedrückt und die Ampulle dabei am Ampullenboden geöffnet, so dass die Monomerflüssigkeit ausfließt.

Dabei kann vorgesehen sein, dass die Hülse der Öffnungseinrichtung an der dem Kartuschenkopf gegenüberliegenden Seite aus der Aufnahme herausragt, wobei vorzugsweise die Hülse dabei derart weit aus der Aufnahme herausragt, dass ein Aufbrechen der Ampulle durch vollständiges Einschieben oder Einschrauben der Hülse in die Aufnahme sichergestellt ist.

Hierdurch kann die Hülse besonders einfach in die Aufnahme gedrückt und dadurch die Ampulle geöffnet werden.

Des Weiteren kann vorgesehen sein, dass die Öffnungseinrichtung eine Verschlusskappe aufweist, die auf die Rückseite der Aufnahme in Richtung des Kartuschenkopfs schraubbar ist, wobei die Verschlusskappe beim Schrauben in Richtung Kartuschenkopf die Hülse in die Aufnahme drückt und dabei die Ampulle im Inneren der Aufnahme öffnet.

Durch das Aufschrauben der Verschlusskappe kann die Hülse mit großer Kraft in die Aufnahme gedrückt werden und dadurch auch eine stabile Ampulle aus Glas geöffnet werden.

Es kann auch vorgesehen sein, dass die Verschlusskappe ein Innengewinde aufweist und die Aufnahme ein dazu passendes rückseitiges Außengewinde und die Verschlusskappe in ihrem Inneren einen Anschlag für die Aufnahme bildet.

Der Abstand zwischen dem Anschlag und dem dem Kartuschenkopf zugewandten Ende des Innengewindes der Verschlusskappe entspricht dem vollen beziehungsweise maximalen Hub des Innengewindes der Verschlusskappe.

Durch diesen Aufbau wird ein besonders kompakter Knochenzementapplikator bereitgestellt, der einfach und zuverlässig bedienbar ist.

Ferner wird mit der vorliegenden Erfindung vorgeschlagen, dass zumindest eine Begasungsöffnung in der Wandung der Aufnahme vorgesehen ist, die das Innere der Aufnahme mit der Umgebung des Knochenzementapplikators verbindet, wobei die zumindest eine Begasungsöffnung durch das Einschieben oder Einschrauben der Öffnungseinrichtung verschließbar ist, insbesondere durch Einschieben oder Einschrauben der Hülse verschließbar ist.

Durch die Begasungsöffnungen kann das Innere der Aufnahme und durch eine Verbindung auch der Innenraum der Kartusche des Knochenzementapplikators mit einem sterilisierenden Gas wie Ethylenoxid sterilisiert werden. Gleichzeitig wird die Begasungsöffnung vor dem Öffnen des Monomerflüssigkeitsbehälters mit der Hülse verschlossen, so dass keine Monomerflüssigkeit durch die Begasungsöffnungen nach außen dringen kann.

Es kann vorgesehen sein, dass das Innere der Aufnahme mit dem Innenraum der Kartusche flüssigkeitsdurchlässig verbunden ist, wobei bevorzugt hierzu die dem Kartuschenkopf zugewandte Vorderseite der Aufnahme wenigstens einen flüssigkeitsdurchlässigen Durchgang und der Austragskolben wenigstens einen flüssigkeitsdurchlässigen Kanal aufweisen.

Hiermit wird sichergestellt, dass die Monomerflüssigkeit ohne weiteres bei geeigneter Aufstellung des Knochenzementapplikators (mit dem Kartuschenkopf nach unten) aus der Aufnahme in den Innenraum der Kartusche zwischen den Austragskolben und den Kartuschenkopf fließen kann.

Bevorzugt ist das Innere der Aufnahme mit dem Innenraum der Kartusche flüssigkeitsdurchlässig aber für das Knochenzementpulver undurchlässig verbunden, wobei besonders bevorzugt der Austragskolben wenigstens einen flüssigkeitsdurchlässigen und für das Knochenzementpulver undurchlässigen Kanal aufweist. Hierzu ist besonders bevorzugt eine Porenscheibe am oder im Austragskolben angeordnet.

Zur einfacheren Montage des Knochenzementapplikators kann vorgesehen sein, dass der Kartuschenkopf ein auf die Kartusche aufschraubbarer Kartuschendeckel ist, wobei der Kartuschendeckel den Innenraum der Kartusche an deren Vorderseite gasdicht und flüssigkeitsdicht abdichtet und wobei die Austragsöffnung in dem Kartuschendeckel angeordnet ist.

Hierdurch kann der Knochenzementapplikator besonders einfach und kostengünstig zusammengebaut werden. Andere Teile des Knochenzementapplikators können so einfach in die ansonsten zylindrische Kartusche eingesetzt werden, bevor der Kartuschenkopf die Kartusche abschließt.

Ferner kann vorgesehen sein, dass die Kartusche an deren Rückseite ein Innengewinde und/oder einen Ring mit einem Innengewinde zum Einschrauben der Aufnahme aufweist, wobei an der Aufnahme ein zu dem Innengewinde der Kartusche oder zu dem Innengewinde des Rings passendes Außengewinde vorgesehen ist.

Hierdurch kann die Aufnahme kraftvoll in dem Innenraum der Kartusche vorangetrieben werden, so dass der Mischstab bequem von der Aufnahme gelöst werden kann.

Des Weiteren kann vorgesehen sein, dass an der ins Innere der Aufnahme weisenden Seite der Aufnahme ein Dorn zum Öffnen des Monomerflüssigkeitsbehälters angeordnet ist.

Hiermit kann der Monomerflüssigkeitsbehälter an einer definierten Stelle innerhalb der Aufnahme geöffnet werden.

Dabei kann vorgesehen sein, dass der Mischstab bis in den Dorn reicht und sich der Mischstab beim Lösen des Mischstabs von der Aufnahme durch den Dorn hindurch drückt oder der Dorn eine Verlängerung des Mischstabs ist und sich beim Lösen des Mischstabs von der Aufnahme auch der Dorn von der Aufnahme trennt.

Durch diese beiden Maßnahmen kann der Mischstab beim Austragen des Knochenzements aus dem Innenraum der Kartusche zuverlässig in die Aufnahme gedrückt werden, ohne dass sich der Mischstab dabei in der Aufnahme verklemmt, wie beispielsweise an Bruchstücken des geöffneten Monomerflüssigkeitsbehälters.

Es kann also vorgesehen sein, dass der Mischstab in die Aufnahme innerhalb eines in das Innere der Aufnahme weisenden Dorns derart angeordnet ist, dass der Mischstab durch den Dorn hindurch in das Innere der Aufnahme drückbar ist.

Hierdurch wird der Mischstab gezielt durch die mit dem Dorn erzeugte Öffnung in dem Monomerflüssigkeitsbehälter in den Monomerflüssigkeitsbehälter eingeschoben. Bevorzugt ist der Mischstab hierzu aus einem härteren Material gefertigt als der Dorn und die Aufnahme. Beispielsweise kann der Mischstab aus Metall bestehen und der Dorn mit der Aufnahme aus einem Kunststoff.

Es kann auch vorgesehen sein, dass der Mischstab in seiner Verbindung zur Aufnahme eine Kreisscheibe mit einem Außengewinde aufweist, wobei die Kreisscheibe in ein passendes Innengewinde an der dem Kartuschenkopf zugewandten Vorderseite der Aufnahme geschraubt ist, wobei bevorzugt das Außengewinde der Kreisscheibe und das Innengewinde an der Vorderseite der Aufnahme Linksgewinde sind.

Hierdurch lässt sich der Mischstab mit der Kreisscheibe von der Vorderseite der Aufnahme durch eine Linksdrehung trennen und der Mischstab mit der Kreisscheibe in das Innere der Aufnahme drücken, wenn die Aufnahme in den Innenraum der Kartusche gedrückt oder geschraubt wird.

Es kann vorgesehen sein, dass der Austragskolben gegen seitlichen Innenwände des Innenraums abgedichtet ist, so dass der Austragskolben gasdicht innerhalb des Innenraums beweglich ist.

Hiermit kann verhindert werden, dass der Knochenzement zwischen dem Austragskolben und der Innenwand der Kartusche aus dem Knochenzementapplikator herausgedrückt werden kann. Zudem kann durch eine Bewegung des Austragskolbens ein Unterdruck im Innenraum der Kartusche erzeugt werden.

Bevorzugt kann auch vorgesehen sein, dass die Aufnahme an ihrer dem Kartuschenkopf gegenüberliegenden Rückseite einen größeren Durchmesser als der Innenraum der Kartusche aufweist.

Hierdurch wird ein Anschlag realisiert, bis zu dem die Aufnahme in den Innenraum der Kartusche bewegt werden kann. Dadurch wird verhindert, dass der Austragskolben mit großer Kraft gegen den Kartuschenkopf gedrückt werden kann und dadurch den Kartuschenkopf über den Mischer deformiert und dabei den Knochenzementapplikator an diesen Stellen ungewollt aufbricht.

Gemäß einer besonders bevorzugten Weiterbildung der vorliegenden Erfindung kann vorgesehen sein, dass an der Rückseite der Kartusche ein Ring mit einem Gewinde, bevorzugt mit einem Innengewinde, angeordnet ist, wobei die Aufnahme ein zum Gewinde des Rings passendes Gegengewinde, insbesondere ein passendes Außengewinde, aufweist und wobei der Ring durch Verschieben oder durch Schrauben in axialer Richtung bezüglich der Zylinderachse des zylindrischen Innenraums der Kartusche gegen die Kartusche beweglich mit der Kartusche verbunden ist.

Hierdurch kann die Aufnahme in das Gewinde des Rings geschraubt werden und so in den Innenraum der Kartusche gedrückt werden, auch wenn der Mischer des Mischstabs im Innenraum an dem Kartuschenkopf anliegt und das Gegengewinde der Aufnahme einen Anschlag für die Aufnahme bildet, indem der Ring weg vom Kartuschenkopf gezogen oder geschraubt wird, so dass das Gegengewinde der Aufnahme in das Gewinde des Rings greift und so in die Kartusche geschraubt werden kann.

Der verschiebbare oder schraubbare Ring dient also dazu, dass die Aufnahme beim Mischen so tief in den Innenraum eingeschoben werden kann, dass die Innenwand des Kartuschenkopfs von dem Mischer abgekratzt wird. Beim Mischen darf das Außengewinde der Aufnahme noch nicht im Eingriff mit dem Gewinde an der Rückseite der Kartusche beziehungsweise des Rings sein, weil sonst ein manuelles Mischen nicht möglich ist. Nach dem Mischen muss jedoch das Gegengewinde der Aufnahme in Eingriff mit dem Gewinde des Rings an der Kartusche oder einer daran befestigten Vorrichtung gebracht werden. Diese Forderung steht eigentlich im Widerspruch mit der freien axialen Beweglichkeit der Aufnahme zum Mischen des Inhalts des Innenraums der Kartusche. Zudem ist für ein Durchstoßen des Mischstabs durch den hohlen Dorn eine relativ hohe Kraft notwendig. Das bedeutet, dass entweder der Anwender zuerst die Aufnahme mit Gewalt gegen den Kartuschenkopf bewegen muss, um den Mischstab durch den Dorn zu stechen, um die Aufnahme in Eingriff mit dem Gewinde an der Rückseite der Kartusche zu bringen, oder es muss eine Vorrichtung vorhanden sein, die nach erfolgtem Mischen eine Verbindung zwischen der Kartusche und der Aufnahme herstellt, so dass die Aufnahme in Eingriff mit dem Gewinde an der Rückseite der Kartusche gebracht wird. Zum "kraftfreien" Verbinden ohne Hammerschlag ist der Ring, der das Gewinde besitzt, auf Kartusche in Längsrichtung verschiebbar oder schraubbar angeordnet.

Der Ring kann als Schiebering oder als Schraubring ausgeführt sein.

Bei Knochenzementapplikatoren mit Ring kann auch vorgesehen sein, dass an der Rückseite der Kartusche ein Anschlag für den Ring vorgesehen ist, über den hinaus der Ring nicht in Richtung vom Kartuschenkopf weg bewegbar ist, so dass der Ring nur einen begrenzten Weg axial in Richtung vom Kartuschenkopf weg verschiebbar oder schraubbar ist.

Hierdurch kann der Ring nicht von der Kartusche gelöst werden und die Aufnahme kann über den Ring als Verbindungsmittel in die Kartusche geschraubt werden.

Der Ring ist vorzugsweise durch Stege gegen Verdrehung gesichert, wenn er verschiebbar an der Kartusche befestigt ist, also wenn er als Schiebering ausgeführt ist. In der Anwendung drückt der Anwender nach erfolgtem Mischen die Aufnahme soweit als möglich gegen den Kartuschenkopf. Anschließend verschiebt er den verschiebbaren Verschiebering oder schraubt den schraubbaren Schraubring in Richtung der Aufnahme. Dann schraubt er die Aufnahme in das Gewinde des Rings ein und schraubt die Aufnahme in Richtung Kartuschenkopf. Zuerst wird der Mischstab durch den hohlen Dorn gestoßen beziehungsweise von der Aufnahme getrennt. Dann drückt die Aufnahme gegebenenfalls auf den separaten Austragskolben mit und presst nach Öffnung des Kartuschenkopfs den Zementteig aus der hierzu geöffneten Austragsöffnung im Kartuschenkopf. Bei der Vorwärtsbewegung der Aufnahme in Richtung des Kartuschenkopfs taucht der Mischstab in die Aufnahme beziehungsweise in den geleerten Monomerflüssigkeitsbehälter ein. Am Ende verschiebt sich der Schiebering in Richtung Kartuschenkopf, bis der Austragskolben an die Rückseite des Mischers anschlägt.

Die zweite Variante mit einem Schraubring (Schraubhülse) funktioniert in derselben Weise. Nur wird hier der Schraubring nach erfolgtem Mischen in Richtung des Ampullenhalters auf dem Außengewinde der Kartusche gedreht und nicht geschoben. Am Ende dreht sich der Schraubring soweit in Richtung des Kartuschenkopfs mit, bis der Kolben mit der Porenscheibe auf die Rückseite des Mischers anschlägt.

Die der vorliegenden Erfindung zugrundeliegenden Aufgaben werden auch gelöst durch ein Verfahren zur Herstellung eines Knochenzements mit einem erfindungsgemäßen Knochenzementapplikator umfassend die folgenden Schritte
A) Öffnen des Monomerflüssigkeitsbehälters im Inneren der Aufnahme und Ausfließen der Monomerflüssigkeit aus dem Monomerflüssigkeitsbehälter, wobei die Monomerflüssigkeit aus der Aufnahme in das Knochenzementpulver im Innenraum der Kartusche fließt,
B) abwechselndes Herausziehen der Aufnahme aus dem Innenraum der Kartusche und Einschieben der Aufnahme in den Innenraum der Kartusche, wobei durch diese Bewegung der Mischer in dem Innenraum der Kartusche bewegt wird und dadurch das Knochenzementpulver und die Monomerflüssigkeit miteinander zum Knochenzement gemischt werden,
C) Lösen des Mischstabs von der Aufnahme durch eine Schraubbewegung oder Drehbewegung der Aufnahme gegen die Kartusche und/oder durch Einpressen der Aufnahme in den Innenraum der Kartusche,
D) Öffnen der Austragsöffnung, und
E) Auspressen des Knochenzements aus dem Innenraum der Kartusche durch die geöffnete Austragsöffnung, wobei der Knochenzement mit dem Austragskolben aus dem Innenraum der Kartusche gepresst wird und der Austragskolben durch Eindrücken oder Einschrauben der Aufnahme in den Innenraum der Kartusche angetrieben wird und wobei der Mischstab in die Aufnahme hineingedrückt wird.

Bei dünnflüssigeren Knochenzementen kann die Aufnahme zuerst aus dem Innenraum der Kartusche herausgezogen werden und dadurch der Mischer zunächst von dem Kartuschenkopf weg in Richtung der Rückseite des Innenraums der Kartusche gezogen werden. Bei hochviskosen Knochenzementen muss zunächst die Aufnahme in den Innenraum der Kartusche eingeschoben werden und dabei der Mischer zunächst von der Rückseite aus in Richtung des Kartuschenkopfs gedrückt werden. Hierdurch wird verhindert, dass sich beim Einleiten der Monomerflüssigkeit im Bereich der Einmündung eine stabile Gelschicht als Reaktionsprodukt des Knochenzementpulvers und der Monomerflüssigkeit bildet, die von nachfließender Monomerflüssigkeit nicht mehr durchdrungen werden kann.

Es kann vorgesehen sein, dass der Austragskolben als separates Teil im Innenraum der Kartusche angeordnet ist und der Austragskolben wenigstens einen für das Knochenzementpulver undurchlässigen und für die Monomerflüssigkeit und Gase durchlässigen Kanal aufweist, wobei in Schritt A) die Monomerflüssigkeit durch den Austragskolben hindurch in den durch den Austragskolben und den Kartuschenkopf begrenzten vorderen Teil des Innenraums der Kartusche fließt, in Schritt B) der Mischstab durch eine Durchführung in dem Austragskolben bewegt wird und in Schritt E) der Austragskolben von der Aufnahme in Richtung des Kartuschenkopfs gedrückt wird.

Hiermit wird sichergestellt, dass das Knochenzementpulver vollständig mit der Monomerflüssigkeit gemischt werden kann.

Dabei kann vorgesehen sein, dass ein in dem Knochenzement enthaltenes Gas in Schritt E) beim Eindrücken oder Einschrauben der Aufnahme in den Innenraum der Kartusche durch den wenigstens einen Kanal im Austragskolben aus dem Knochenzement herausgepresst wird.

Hierdurch wird der Knochenzement beim Auspressen durch den Austragskolben entgast.

Ferner kann vorgesehen sein, dass der Monomerflüssigkeitsbehälter in Schritt A) durch das Einschieben oder Einschrauben einer Öffnungseinrichtung in die Aufnahme geöffnet wird.

Hiermit wird das Verfahren für den Anwender besonders einfach umsetzbar. Zudem kann so eine definierte Kraft zum Öffnen des Monomerflüssigkeitsbehälters bereitgestellt werden und damit ein reproduzierbares Öffnen des Monomerflüssigkeitsbehälters erreicht werden.

Dabei kann vorgesehen sein, dass der Monomerflüssigkeitsbehälter in Schritt A) auf einen Dorn im Inneren der Aufnahme gedrückt wird und dadurch in der Aufnahme geöffnet wird, wobei vorzugsweise der Monomerflüssigkeitsbehälter eine Ampulle aus Glas oder einem Kunststoff ist und die Ampulle mit dem Dorn an einem Ampullenboden der Ampulle geöffnet wird.

Auch dies dient dazu, den Monomerflüssigkeitsbehälter an einer definierten Stelle zu öffnen und dadurch den Öffnungsvorgang des Monomerflüssigkeitsbehälters reproduzierbar zu machen.

Dabei kann wiederum vorgesehen sein, dass in Schritt E) der Dorn mit dem Mischstab in die Aufnahme gedrückt wird oder der Mischstab den Dorn durchstößt und durch den Dorn hindurch in die Aufnahme gedrückt wird.

Hiermit wird sichergestellt, dass der Mischstab ohne Widerstand durch den geöffneten Monomerflüssigkeitsbehälter oder durch dessen Bruchstücke in die Aufnahme und in den geöffneten Monomerflüssigkeitsbehälter hinein drückbar ist.

Des Weiteren kann vorgesehen sein, dass die Aufnahme in Schritt B) linear bewegt wird und in Schritt C) und E) in die Kartusche eingeschraubt wird, wobei die lineare Bewegung in Schritt A) durch ein Gewinde an der Aufnahme als Anschlag begrenzt wird, wobei das Gewinde in den Schritten C) und E) zum Einschrauben der Aufnahme in die Kartusche genutzt wird.

Hiermit kann verhindert werden, dass der Mischstab bereits beim Mischen von der Aufnahme gelöst wird. Ferner wird so auch verhindert, dass bereits beim Mischen eine große Kraft auf den Verschluss der Austragsöffnung ausgeübt wird und auch dass bereits vor dem Abschluss des Mischvorgangs Knochenzement aus der Kartusche austritt. Zudem kann so der Knochenzement durch das Schrauben kraftvoll aus dem Innenraum der Kartusche ausgetrieben werden.

Bevorzugte Ausgestaltungen des erfindungsgemäßen Verfahrens können vorsehen, dass das Innere der Aufnahme vor Schritt A) mit der Umgebung des Knochenzementapplikators gasdurchlässig verbunden ist, wobei das Innere der Aufnahme vor Schritt A) oder während Schritt A) beim Öffnen des Monomerflüssigkeitsbehälters verschlossen wird.

Hiermit kann das Innere der Aufnahme und auch der Innenraum der Kartusche, also der gesamte Knochenzementapplikator einschließlich seines Inhalts mit einem sterilisierenden Gas wie Ethylenoxid sterilisiert werden. Gleichzeitig kann die Monomerflüssigkeit nicht aus der Aufnahme austreten, nachdem der Monomerflüssigkeitsbehälter innerhalb der Aufnahme geöffnet wurde.

Es kann auch vorgesehen sein, dass der Knochenzementapplikator vor Schritt A) mit dem Kartuschenkopf nach unten gehalten oder aufgestellt wird, wobei vorzugsweise der Kartuschenkopf zumindest während der Schritte A) und B) nach unten ausgerichtet bleibt, so dass die Monomerflüssigkeit getrieben von der Schwerkraft in den Innenraum der Kartusche fließt.

Hierdurch wird keine zusätzliche Pumpe benötigt, um die Monomerflüssigkeit in den Innenraum der Kartusche zum Knochenzementpulver zu transferieren.

Des Weiteren kann vorgesehen sein, dass beim Einschieben der Aufnahme in den Innenraum der Kartusche während Schritt B) ein bis dahin zurückgebliebener Teil der Monomerflüssigkeit in den Innenraum der Kartusche gedrückt wird.

Hierdurch wird erreicht, dass die Monomerflüssigkeit möglichst vollständig in das Knochenzementpulver transferiert wird, um das gewünschte Mischungsverhältnis von Knochenzementpulver zu Monomerflüssigkeit zu erhalten und damit einen Knochenzement mit den gewünschten Eigenschaften zu erzeugen.

Zudem kann vorgesehen sein, dass vor Schritt C) die Aufnahme vollständig in den Innenraum der Kartusche eingeschoben wird, so dass der Mischer im Innenraum der Kartusche am Kartuschenkopf anliegt, wobei in Schritt C) der Mischstab durch weiteres Eindrücken oder Einschrauben der Aufnahme in die Kartusche von der Aufnahme gelöst wird und in Schritt E) die Aufnahme hinein gedrückt wird.

Hiermit kann der Mischstab auf einfache Weise kraftvoll von der Aufnahme gelöst werden.

Schließlich kann vorgesehen sein, dass ein Ring mit einem Gewinde an der Rückseite der Kartusche angeordnet ist und die Aufnahme ein passendes Gegengewinde aufweist, wobei hach Schritt B) und vor Schritt C) der Ring in axialer Richtung bezüglich des zylindrischen Innenraums der Kartusche vom Kartuschenkopf weg geschoben oder geschraubt wird, so dass das Gegengewinde der Aufnahme in das Gewinde des Rings greift und während der Schritte C) und E) die Aufnahme in das Gewinde des Rings geschraubt wird und dabei die Aufnahme im Innenraum der Kartusche in Richtung des Kartuschenkopfs bewegt wird, so dass in Schritt C) der Mischstab, der an der Innenseite des Kartuschenkopfs anliegt, von der Aufnahme gelöst wird und in Schritt E) der gelöste Mischstab in die Aufnahme gedrückt wird und der Austragskolben mit der Vorderseite der Aufnahme in Richtung des Kartuschenkopfs gedrückt wird.

Hierdurch ergeben sich die oben bei dem Knochenzementapplikator mit dem Ring genannten Vorteile.

Der Erfindung liegt die überraschende Erkenntnis zugrunde, dass es durch den von der Aufnahme lösbaren Mischstab und den in die Aufnahme versenkbaren Mischstab gelingt, einen Knochenzementapplikator bereitzustellen, bei dem der Mischstab nicht aus dem Knochenzementapplikator herausgezogen werden muss und bei dem der Mischstab nicht abgebrochen und entfernt werden muss, wenn der Knochenzement mit dem Knochenzementapplikator ausgetragen wird. Die Aufnahme, in der der Monomerflüssigkeitsbehälter angeordnet ist, kann dabei überraschend zur Aufnahme des Mischstabs verwendet werden. So behindert der Mischstab nicht die Bewegung des Austragskolbens beim Auspressen des Knochenzements. Des Weiteren besteht dadurch, dass die Aufnahme im rückseitigen Teil des Innenraums bewegt wird, die Möglichkeit, die Vorderseite der Aufnahme als Austragskolben zu verwenden oder zumindest den Austragskolben direkt mit der Aufnahme anzutreiben. Dadurch kann eine axiale Bewegung der Aufnahme sowohl zum Mischen des Knochenzements im Innenraum der Kartusche verwendet werden als auch zum Austreiben des Knochenzements beziehungsweise zum Antreiben des Austragskolbens.

Der Mischstab wird nach der Öffnung des Monomerflüssigkeitsbehälters in den hohlen, von der Monomerflüssigkeit geleerten Monomerflüssigkeitsbehälter innerhalb der Aufnahme geschoben, da der Mischstab und der Monomerflüssigkeitsbehälter hintereinander in dem Knochenzementapplikator angebracht sind. Der erfindungsgemäße Knochenzementapplikator ist ein Prepack-Mischsystem und kann ohne vorherige Montageschritte bedient werden. Es ist keine äußere Vakuumquelle für den Monomertransfer notwendig. Der Austrag des Knochenzements erfolgt durch eine manuelle Schraubbewegung der hohlzylinderförmigen Aufnahme, die einen Austragskolben an ihrer dem Kartuschenkopf zugewandten Vorderseite bildet oder die einen Austragskolben im Innenraum der Kartusche antreibt. Die Schraubbewegung entwickelt genügend Auspresskraft, um auch einen hochviskosen Knochenzement aus der Kartusche auspressen zu können und auch um den Mischstab von der Aufnahme zu lösen. Die Bauteile des Knochenzementapplikators können im Wesentlichen durch Kunststoffspritzgießen hergestellt werden und bestehen vorzugsweise aus kostengünstigem thermoplastischem Kunststoff. Die O-Ringe bestehen aus in der Medizintechnik üblichen Elastomeren, wie Silikon oder EPDM (Terpolymere aus Ethylen, Propylen und einem Dien).

Ein beispielhafter erfindungsgemäßer Knochenzementapplikator, der zum Lagern, Mischen und Applizieren vorgesehen ist, ist zusammengesetzt aus
a) einer hohlzylinderförmigen Kartusche, wobei an einem vorderen Ende der Kartusche ein Befestigungsmittel für einen Kartuschendeckel (als Kartuschenkopf) angeordnet ist und wobei am entgegengesetzten rückseitigen Ende der Kartusche an der Kartuscheninnenwand ein Innengewinde angeordnet ist,
b) einem Kartuschendeckel, der mit dem Befestigungsmittel am vorderen Ende der Kartusche gasdicht und flüssigkeitsdicht verbunden ist, wobei der Kartuschendeckel eine Austragsöffnung besitzt,
c) einem Verschlussstopfen, der in der Austragsöffnung des Kartuschendeckels gasdicht und lösbar angeordnet ist,
d) einem hohlzylinderförmigen Ampullenhalter als Aufnahme, der an seiner Mantelfläche mindestens in einem ersten Abschnitt ein Außengewinde und in einem zweiten Abschnitt kein Gewinde besitzt,
e) einem Verschluss an der Vorderseite des Ampullenhalters, der den hohlzylinderförmigen Ampullenhalter an einer Längsseite verschließt, wobei ein Mischstab mit einem Mischer an der dem Kartuschenkopf zugewandten Seite des Verschlusses lösbar angebracht ist und wobei die gegenüberliegende Seite des Verschlusses mit einem Stechdorn verbunden ist,
f) einem axial in der Kartusche verschiebbaren Austragskolben, der für Gase und Flüssigkeiten permeabel und für Knochenzementpulverpartikel impermeabel ist, der zwischen dem Mischer und dem Verschluss des Ampullenhalters in der Kartusche angeordnet ist,
g) einem Monomerflüssigkeitsbehälter mit Monomerflüssigkeit, der mit seiner Bodenseite über dem Stechdorn beabstandet in dem Ampullenhalter angeordnet ist,
h) einer verschiebbaren Hülse als Teil einer Öffnungseinrichtung, die oberhalb des Monomerflüssigkeitsbehälters in dem hohlzylinderförmigen Ampullenhalter derart axial verschiebbar angeordnet ist, dass die Öffnungseinrichtung mit der Hülse über den Rand des hohlzylinderförmigen Ampullenhalters herausragt,
i) einer einseitig verschlossenen hohlen Verschlusskappe des hohlzylinderförmigen Ampullenhalters, wobei ein Innengewinde und ein Anschlag für den hohlzylinderförmigen Ampullenhalter in der hohlen Verschlusskappe angeordnet sind, wobei vorzugsweise der Abstand zwischen der unteren Außenkante der Verschlusskappe und dem Anschlag kleiner ist als der Abstand zwischen dem äußeren Ende der Hülse und dem Rand der Schmalseite des Ampullenhalters aus dem die Hülse herausragt,
j) optional mindestens einer Belüftungsöffnung in der Mantelfläche des hohlzylinderförmigen Ampullenhalters, wobei die Belüftungsöffnung durch axiale Verschiebung der Hülse gasdicht verschließbar ist,
k) Knochenzementpulver, das in einem vorderen Teil des Innenraums der Kartusche angeordnet ist, der durch die Innenwand der Kartusche, den Kartuschendeckel und den Austragskolben gebildet wird,
l) wobei der hohlzylinderförmige Ampullenhalter mit seinem Außengewinde mit dem Innengewinde der Kartusche verschraubt ist oder verschraubbar ist und
m) zumindest der Mischstab nach Öffnung des Monomerflüssigkeitsbehälters in den Hohlraum des Ampullenhalters oder des Monomerflüssigkeitsbehälters verschiebbar ist.

Vorteilhaft ist es, wenn der Verschluss mit dem Mischstab und dem Stechdorn einteilig ausgebildet ist. Dadurch kann der Montageaufwand im Vergleich zu einem zweiteiligen oder dreiteiligen Verschluss mit Mischstab und Stechdorn deutlich gesenkt werden. Der einteilige Verschluss mit Mischstab und Stechdorn kann vorteilhaft durch Kunststoffspritzgießen gefertigt werden.

Es kann auch vorgesehen sein, dass der Verschluss in dem hohlzylinderförmigen Ampullenhalter durch Presspassung lösbar fixiert ist. Der Verschluss kann dabei konisch sein und in einem konischen Sitz des hohlzylinderförmigen Ampullenhalters gelagert sein. Der Konus des Verschlusses verjüngt sich in Richtung des Kartuschenkopfs. Bei Bewegung des hohlzylinderförmigen Ampullenhalters in Richtung des Kartuschenkopfs stützt sich der Mischstab mit Mischelementen auf der Innenseite des Kartuschendeckels ab und drückt den konischen Verschluss aus seinem Sitz. Der Stechdorn mit dem Verschluss und dem Mischstab treten dann in das Innere des Ampullenhalters und in den geöffneten Monomerflüssigkeitsbehälter ein.

In einer anderen beispielhaften Ausgestaltungsvariante besitzt ein innerer Teil des Verschlusses in dem hohlzylinderförmigen Ampullenhalter ein Außengewinde, das in ein Innengewinde des hohlzylinderförmigen Ampullenhalters eingeschraubt ist, wobei der innere Teil des Verschlusses bevorzugt ein linksgängiges Außengewinde besitzt. Beim Drehen des hohlzylinderförmigen Ampullenhalters bewegt sich dieser in Richtung Kartuschenkopf. Der Mischstab mit den Mischelementen wird an die Innenseite des Deckels gepresst. Mit steigendem Anpressdruck an den Deckel kann der Mischstab sich nicht mehr mit dem Ampullenhalter mitdrehen. Der innere Teil des Verschlusses wird dadurch aus dem Innengewinde des hohlzylinderförmigen Ampullenhalters gedreht. Der innere Teil des Verschlusses verlässt seinen Sitz in dem hohlzylinderförmigen Ampullenhalter und wird zusammen mit dem Stechdorn und dem Mischstab in den geöffneten Monomerflüssigkeitsbehälter geschoben.

In einer anderen Ausgestaltungsvariante ist der Mischstab in den Verschluss eingepresst und durchdringt nach Öffnung des Monomerflüssigkeitsbehälters den Verschluss und den Stechdorn. Danach wird der Mischstab in den Ampullenhalter eingeschoben.

Es kann ferner vorgesehen sein, dass der hohlzylinderförmige Ampullenhalter an seiner zylinderförmigen Kopfseite einen Durchmesser gleich oder kleiner dem Innendurchmesser der hohlzylinderförmigen Kartusche hat und der hohlzylinderförmige Ampullenhalter mit seiner Kopfseite gasdicht axial in der Kartusche bewegt werden kann.

Es kann ferner vorgesehen sein, dass die Hülse als Hohlzylinder ausgebildet ist, wobei der Zylindermantel der Hülse auf dem Monomerflüssigkeitsbehälter aufliegt und wobei die Hülse im Inneren des Hohlraums oder am Ende der Hülse durch eine gasdichte Trennwand verschlossen ist.

Des Weiteren kann vorgesehen sein, dass der innere Teil des Verschlusses einen kleineren Außendurchmesser besitzt als der Innendurchmesser des Monomerflüssigkeitsbehälters. Dadurch kann der innere Teil des Verschlusses mit dem Stechdorn und dem Mischstab problemlos in das Innere des geöffneten Monomerflüssigkeitsbehälters geschoben werden.

Ein beispielhaftes erfindungsgemäßes Verfahren zur Vermischung und Applizieren von Polymethylmethacrylat-Knochenzement mit einem erfindungsgemäßen Knochenzementapplikator kann beispielsweise mit den folgenden nacheinander ablaufenden Schritten realisiert werden:
a) senkrechte Positionierung des Knochenzementapplikators mit dem Kartuschenkopf nach unten,
b) Schrauben der auf den hohlzylinderförmige Ampullenhalter als Aufnahme angeschraubten Verschlusskappe in Richtung Kartuschenkopf,
c) Verschieben der Hülse in Richtung Kartuschenkopf durch die Verschlusskappe,
d) Optional: Verschluss der mindestens einen Begasungsöffnung in dem hohlzylinderförmigen Ampullenhalter durch die Hülse,
e) Verschieben des Monomerflüssigkeitsbehälters in Richtung Stechdorn durch die axiale Verschiebung der Hülse,
f) Zerstörung des Bodens des Monomerflüssigkeitsbehälters durch den Stechdorn,
g) Ausfließen der Monomerflüssigkeit durch den Verschluss und den für Gase und Flüssigkeiten permeablen Austragskolben in den Innenraum der Kartusche zum Knochenzementpulver,
h) Zurückziehen des hohlzylinderförmigen Ampullenhalters entgegengesetzt zum Kartuschenkopf bei gleichzeitiger Rückwärtsbewegung des Mischstabs unter Mischung des Knochenzementpulvers mit der Monomerflüssigkeit,
i) Vorwärtsbewegung des Ampullenhalters, Transfer der restlichen Monomerflüssigkeit durch den Überdruck oberhalb der Monomerflüssigkeit durch den Verschluss und den für Gase und Flüssigkeiten permeablen Austragskolben, bei gleichzeitiger Rückwärtsbewegung des Mischstabs unter Mischung des Knochenzementpulvers mit der Monomerflüssigkeit,
j) Mehrmaliges Wiederholen der Schritte h) und i)
k) Bildung des Knochenzements aus der Mischung des Polymethylmethacrylat-Knochenzementpulvers mit der Monomerflüssigkeit,
l) Entfernen des Verschlussstopfens aus der Austragsöffnung,
m) Schrauben des hohlzylinderförmigen Ampullenhalters in Richtung des Kartuschenkopfs, wobei der Mischstab mit den Mischelementen auf der Innenseite des Deckels (des Kartuschenkopfs) aufsetzt und den inneren Teil des Verschlusses aus seinem konischen Sitz in dem hohlzylinderförmigen Ampullenhalter in Richtung Kartuschenboden herausdrückt,
n) Einschieben des Verschlusses mit Stechdorn und Mischstab in den geöffneten Monomerflüssigkeitsbehälter, und
o) Herauspressen des Polymethylmethacrylat-Knochenzements durch die Schraubbewegung des Ampullenhalters in Richtung Kartuschenkopf.

Ein beispielhaftes alternatives Verfahren zur Vermischung und Applizieren von Polymethylmethacrylat-Knochenzement mit dem erfindungsgemäßen Knochenzementapplikator kann durch folgende nacheinander ablaufende Schritte charakterisiert sein:
a) senkrechte Positionierung des Knochenzementapplikators mit dem Kartuschenkopf nach unten,
b) Schrauben der auf den hohlzylinderförmigen Ampullenhalter als Aufnahme angeschraubten Verschlusskappe in Richtung Kartuschenkopf,
c) Verschieben der Hülse in Richtung Kartuschenkopf durch die Verschlusskappe,
d) Verschluss der mindestens einen Begasungsöffnung in dem hohlzylinderförmigen Ampullenhalter durch die Hülse,
e) Verschieben des Monomerflüssigkeitsbehälters in Richtung Stechdorn durch die axiale Verschiebung der Hülse,
f) Zerstörung des Bodens des Monomerflüssigkeitsbehälters durch den Stechdorn,
g) Ausfließen der Monomerflüssigkeit durch den Verschluss und den für Gase und Flüssigkeiten permeablen Austragskolben in den vorderen Teil des Innenraums der Kartusche zum Knochenzementpulver,
h) Zurückziehen des hohlzylinderförmigen Ampullenhalters entgegengesetzt zum Kartuschenkopf bei gleichzeitiger Rückwärtsbewegung des Mischstabs unter Mischung des Knochenzementpulvers mit der Monomerflüssigkeit,
i) Vorwärtsbewegung des Ampullenhalters, Transfer der restlichen Monomerflüssigkeit durch den Überdruck oberhalb der Monomerflüssigkeit durch den Verschluss und den für Gase und Flüssigkeiten permeablen Austragskolben, bei gleichzeitiger Rückwärtsbewegung des Mischstabs unter Mischung des Knochenzementpulvers mit der Monomerflüssigkeit,
j) Mehrmaliges Wiederholen der Schritte h) und i),
k) Bildung des Knochenzements aus der Mischung des Polymethylmethacrylat-Knochenzementpulvers mit der Monomerflüssigkeit,
l) Entfernen des Verschlussstopfens aus der Austragsöffnung,
m) Schrauben des hohlzylinderförmigen Ampullenhalters in Richtung des Kartuschenkopfs, wobei der Mischstab mit den Mischelementen auf der Innenseite des Deckels aufsetzt und den inneren Teil des Verschlusses mit seinem Außengewinde aus dem Innengewinde des hohlzylinderförmigen Ampullenhalters in Richtung Kartuschenboden herausschraubt,
n) Einschieben des Verschlusses mit Stechdorn und Mischstab in den geöffneten Monomerflüssigkeitsbehälter, und
o) Herauspressen des Polymethylmethacrylat-Knochenzements durch die Schraubbewegung des Ampullenhalters in Richtung Kartuschenkopf.

Ein weiteres beispielhaftes alternatives Verfahren zur Vermischung und Applizieren von Polymethylmethacrylat-Knochenzement mit dem erfindungsgemäßen Knochenzementapplikator kann durch folgende nacheinander ablaufende Schritte charakterisiert sein:
a) senkrechte Positionierung des Knochenzementapplikators mit dem Kartuschenkopf nach unten,
b) Schrauben der auf die hohlzylinderförmige Aufnahme angeschraubten Verschlusskappe in Richtung Kartuschenkopf,
c) Verschieben der Hülse in Richtung Kartuschenkopf durch die Verschlusskappe,
d) Verschluss der mindestens einen Begasungsöffnung in der hohlzylinderförmigen Aufnahme durch die Hülse,
e) Verschieben des Monomerflüssigkeitsbehälters in Richtung Stechdorn durch die axiale Verschiebung der Hülse,
f) Zerstörung des Bodens des Monomerflüssigkeitsbehälters durch den Stechdorn,
g) Ausfließen der Monomerflüssigkeit durch den Verschluss und den für Gase und Flüssigkeiten permeablen Austragskolben in den vorderen Teil des Innenraums der Kartusche zum Knochenzementpulver,
h) Zurückziehen der hohlzylinderförmigen Aufnahme entgegengesetzt zum Kartuschenkopf bei gleichzeitiger Rückwärtsbewegung des Mischstabs unter Mischung des Knochenzementpulvers mit der Monomerflüssigkeit,
i) Vorwärtsbewegung der Aufnahme, Transfer der restlichen Monomerflüssigkeit durch den Überdruck oberhalb der Monomerflüssigkeit durch den Verschluss und den für Gase und Flüssigkeiten permeablen Austragskolben, bei gleichzeitiger Rückwärtsbewegung des Mischstabs unter Mischung des Knochenzementpulvers mit der Monomerflüssigkeit,
j) Mehrmaliges Wiederholen der Schritte h) und i),
k) Bildung des Knochenzements aus der Mischung des Polymethylmethacrylat-Knochenzementpulvers mit der Monomerflüssigkeit,
l) Entfernen des Verschlussstopfens aus der Austragsöffnung,
m) Schrauben der hohlzylinderförmigen Aufnahme in Richtung des Kartuschenkopfs, wobei der Mischstab mit den Mischelementen auf der Innenseite des Deckels aufsetzt und den Verschluss und den Stechdorn durchsticht,
n) Einschieben des Mischstabs in den geöffneten Monomerflüssigkeitsbehälter, und
o) Herauspressen des Polymethylmethacrylat-Knochenzements durch die Schraubbewegung der Aufnahme in Richtung Kartuschenkopf.

Das Auspressen des Knochenzements erfolgt durch Antreiben des Austragskolbens mit der Aufnahme beziehungsweise mit dem Ampullenhalter.

Im Folgenden werden weitere Ausführungsbeispiele der Erfindung anhand von zwölf schematisch dargestellten Figuren erläutert, ohne jedoch dabei die Erfindung zu beschränken. Dabei zeigt:
Figur 1: eine schematische Querschnittansicht eines beispielhaften ersten erfindungsgemäßen Knochenzementapplikators zum Herstellen eines Knochenzementteigs;
Figur 2: eine schematische perspektivische Außenansicht des ersten erfindungsgemäßen Knochenzementapplikators nach Figur 1;
Figur 3: eine schematische Seitenansicht des ersten erfindungsgemäßen Knochenzementapplikators nach den Figuren 1 und 2;
Figur 4: eine schematische Querschnittansicht des ersten erfindungsgemäßen Knochenzementapplikators nach den Figuren 1 bis 3 mit geöffnetem Monomerflüssigkeitsbehälter zur Verdeutlichung des Ablaufs eines erfindungsgemäßen Verfahrens;
Figur 5: eine schematische Querschnittansicht des ersten erfindungsgemäßen Knochenzementapplikators nach den Figuren 1 bis 4 mit in die Kartusche eingeschobener Aufnahme zur Verdeutlichung des Ablaufs eines erfindungsgemäßen Verfahrens;
Figur 6: eine schematische Querschnittansicht des ersten erfindungsgemäßen Knochenzementapplikators nach den Figuren 1 bis 5 mit in die Kartusche eingeschraubter Aufnahme nach Austragen des Knochenzements zur Verdeutlichung des Ablaufs eines erfindungsgemäßen Verfahrens;
Figur 7: eine schematische perspektivische Außenansicht eines beispielhaften zweiten erfindungsgemäßen Knochenzementapplikators zum Herstellen eines Knochenzementteigs;
Figur 8: eine schematische perspektivische Querschnittansicht des zweiten erfindungsgemäßen Knochenzementapplikators nach Figur 7 ohne die Ausgangskomponenten im Lagerungszustand;
Figur 9: eine schematische perspektivische Querschnittansicht des zweiten erfindungsgemäßen Knochenzementapplikators nach den Figur 7 und 8 ohne die Ausgangskomponenten mit geöffnetem Monomerflüssigkeitsbehälter zur Verdeutlichung des Ablaufs eines erfindungsgemäßen Verfahrens;
Figur 10: eine schematische Querschnittansicht des zweiten erfindungsgemäßen Knochenzementapplikators nach Figur 9 mit geöffnetem Monomerflüssigkeitsbehälter;
Figur 11: eine schematische Querschnittansicht des zweiten erfindungsgemäßen Knochenzementapplikators nach den Figuren 7 bis 11 mit in die Kartusche eingeschobener Aufnahme zur Verdeutlichung des Ablaufs eines erfindungsgemäßen Verfahrens; und
Figur 12: eine schematische Querschnittansicht des zweiten erfindungsgemäßen Knochenzementapplikators nach den Figuren 7 bis 11 mit in die Kartusche eingeschraubter Aufnahme nach Austragen des Knochenzements zur Verdeutlichung des Ablaufs eines erfindungsgemäßen Verfahrens.

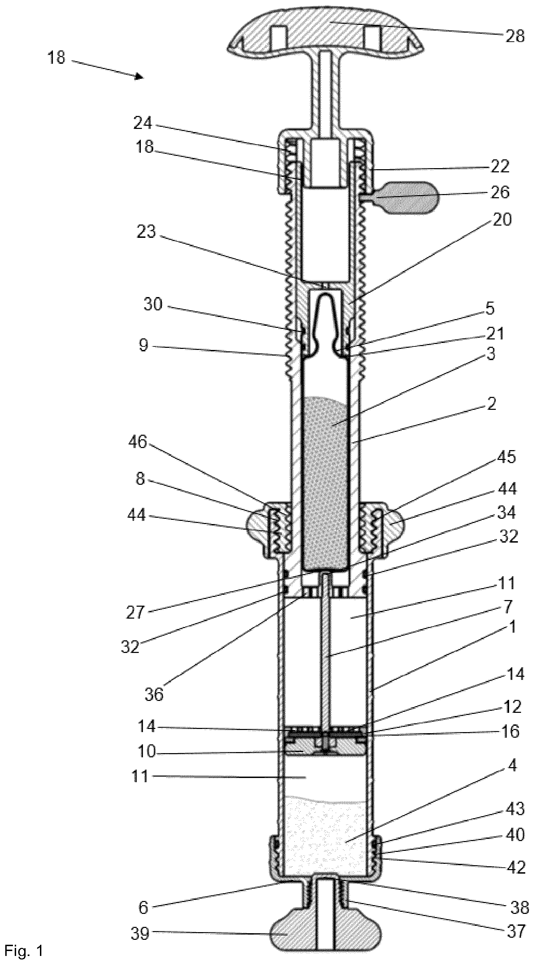

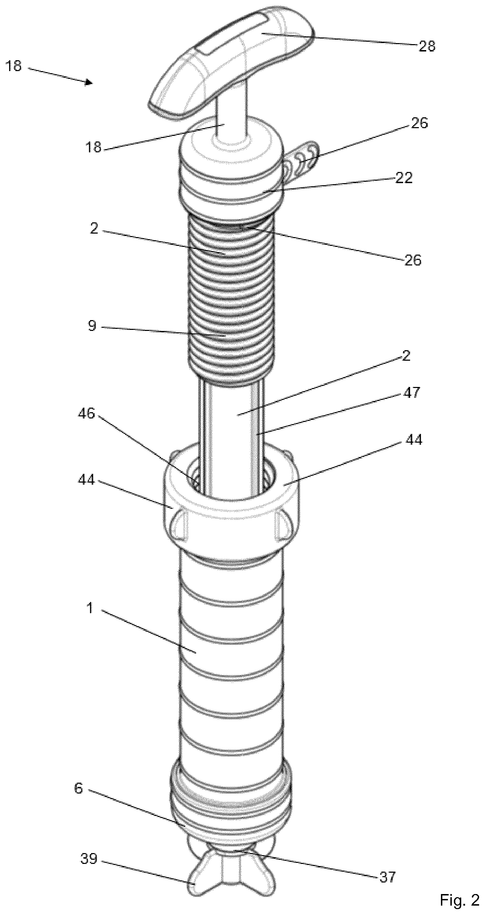

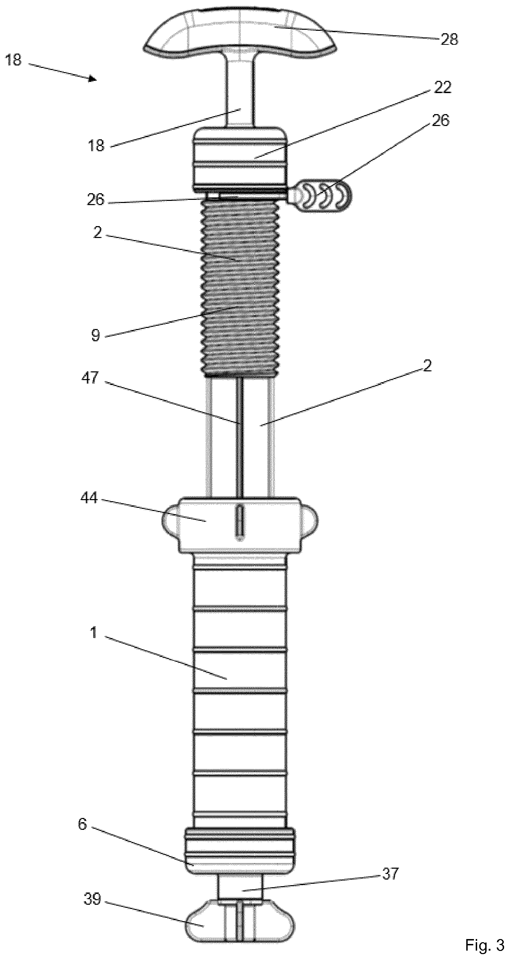

In den Figuren 1 bis 6 sind Abbildungen eines ersten erfindungsgemäßen Knochenzementapplikators zum Lagern von Ausgangskomponenten 3, 4 eines Knochenzements 48 und zum Mischen des Knochenzements 48 gezeigt. Die Figuren 1 und 4 bis 6 zeigen dabei den Ablauf eines erfindungsgemäßen Verfahrens durchgeführt mit dem ersten erfindungsgemäßen Knochenzementapplikator in Form von vier Querschnittansichten des ersten erfindungsgemäßen Knochenzementapplikators.

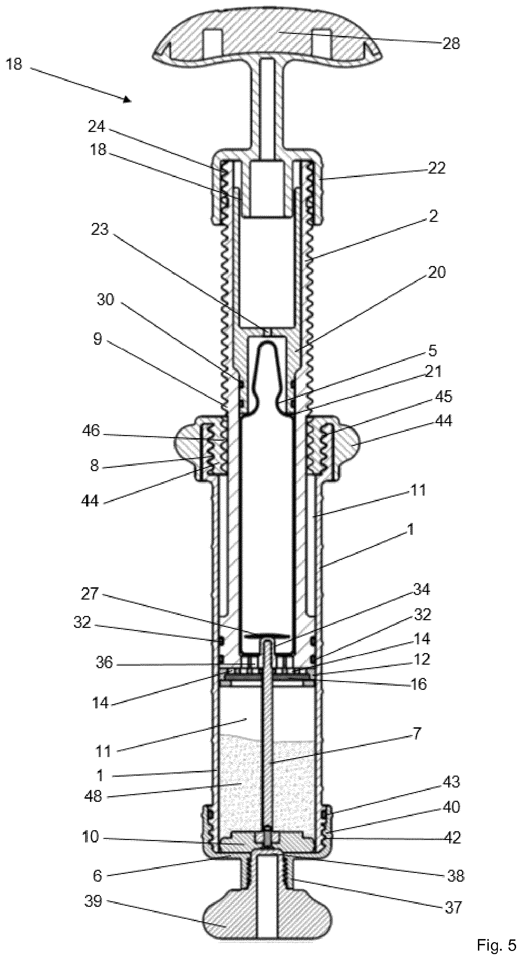

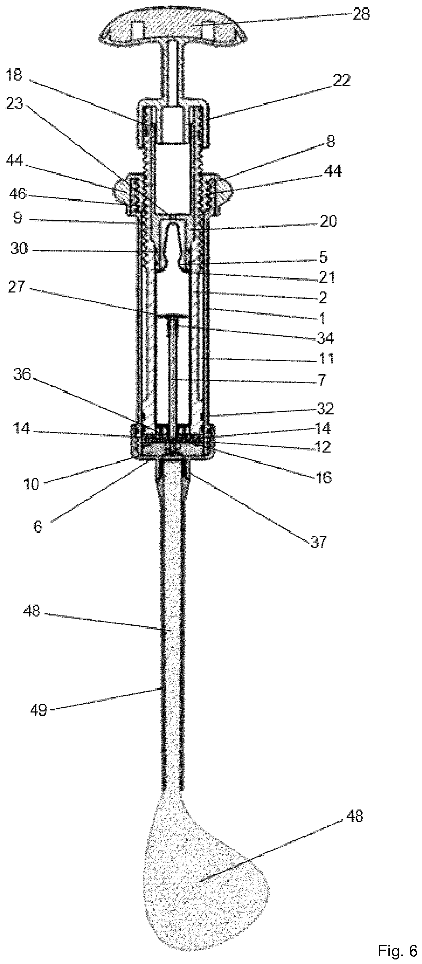

Der erste erfindungsgemäße Knochenzementapplikator weist eine röhrenförmige Kartusche 1 aus Kunststoff auf, die einen vorderen Teil (in den Figuren 1 bis 6 unten) des Knochenzementapplikators bildet. Ein rückseitiger, hinterer Teil des Knochenzementapplikators wird durch eine Aufnahme 2 gebildet. Der Knochenzementapplikator ist zur Herstellung eines Knochenzements 48 (siehe Figuren 5 und 6) vorgesehen, der aus einer Monomerflüssigkeit 3 und aus einem Knochenzementpulver 4 hergestellt wird. Die Monomerflüssigkeit 3 und das Knochenzementpulver 4 sind die Ausgangskomponenten 3, 4 des Knochenzements 48. Die Monomerflüssigkeit 3 ist in einer aufbrechbaren Ampulle 5 aus Glas oder aus einem Kunststoff als Monomerflüssigkeitsbehälter für die Monomerflüssigkeit 3 enthalten, wobei die Ampulle 5 in der Aufnahme 2 steckt. Die Kartusche 1 bildet in ihrem Inneren einen zylindrischen Innenraum 11, in dem das Knochenzementpulver 4 enthalten ist. Somit ist der Knochenzementapplikator auch zum Lagern der Monomerflüssigkeit 3 und des Knochenzementpulvers 4 geeignet.

Die Kartusche 1 weist an ihrer Vorderseite (in den Figuren unten) einen Kartuschendeckel 6 als Kartuschenkopf auf. In dem Kartuschendeckel 6 ist eine Austragsöffnung vorgesehen. In der seitlichen Wandung der Aufnahme 2 können sich gemäß einer alternativen Variante des Knochenzementapplikators mehrere Begasungsöffnungen (nicht gezeigt) befinden, durch die ein Gas aus dem Inneren des Knochenzementapplikators abgesaugt werden kann und durch die ein sterilisierendes Gas wie Ethylenoxid eingefüllt werden kann, um das Innere des Knochenzementapplikators zu sterilisieren.

An der Vorderseite der Aufnahme 2 ist ein Mischstab 7 befestigt, der sich von der Vorderseite der Aufnahme 2 bis in den vorderen Teil der Kartusche 1 erstreckt, in dem sich das Knochenzementpulver 4 befindet.

Am rückseitigen Ende der Kartusche 1 befindet sich ein Innengewinde 8. Die Aufnahme 2 weist an ihrer Außenseite ein Außengewinde 9 auf, das einen kleineren Durchmesser als das Innengewinde 8 der Kartusche 1 hat. Die Aufnahme 2 ist in einem hinteren Bereich nach Art eines Gewinderohrs geformt und weist in ihrem Inneren eine zylindrische Kammer auf, in der die Ampulle 5 steckt. In einem vorderen Bereich ist die Aufnahme 2 außen zylindrisch geformt, wobei vier vorspringende Leisten 47 parallel zur Zylinderachse der Aufnahme 2 an der äußeren Oberfläche der Aufnahme 2 vorgesehen sind. Die Ampulle 5 hat einen zylindrischen Ampullenkörper mit einem zu dem Inneren der Aufnahme 2 passendem Durchmesser. Im Inneren der Kartusche 1 bildet die Kartusche 1 den zylindrischen Innenraum 11. Die Zylindergeometrie des Innenraums 11 und der Kammer der Aufnahme 2 entsprechen Zylindern mit kreisförmiger Grundfläche.

An der Vorderseite des Mischstabs 7 ist ein Mischer 10 in Form von Mischflügeln mit einem umgebenden Abkratzring befestigt. Mit dem Abkratzring können die Bereiche direkt an der Innenwand des Innenraums 11 erreicht werden.

Die Aufnahme 2 ist an ihrer Vorderseite durch eine Wandung mit mehreren Durchgängen 36 als Verschluss der Vorderseite begrenzt, wobei die Wandung an der Vorderseite der Aufnahme 2 die Kammer nach vorne an ihrer kreisförmigen Grundfläche verschließt. Im Innenraum 11 der Kartusche 1 ist ein in axialer Richtung des zylindrischen Innenraums 11 beweglicher Austragskolben 12 angeordnet, der in Presspassung in dem Innenraum 11 angeordnet ist. Der Mischstab 7 ist durch einen zentralen Durchgang im Austragskolben 12 geführt, so dass der Mischstab 7 gegen den Austragskolben 12 bewegt werden kann, ohne dass sich dabei der Austragskolben 12 im Innenraum der Kartusche 1 bewegt. Bei zurückgezogener Aufnahme 2 liegt der Mischer 10 an der Vorderseite des Austragskolbens 12 an. Dadurch kann der gesamte vordere Teil des Innenraums 11, der seitlich durch die Kartusche 1, vorne durch den Kartuschendeckel 6 und hinten durch den Austragskolben 12 begrenzt ist, von dem Mischer 10 erreicht werden. Dadurch ist eine vollständige Durchmischung des Knochenzementpulvers 4 mit der Monomerflüssigkeit 3 in diesem Bereich gewährleistet.

Der Austragskolben 12 weist mehrere Kanäle 14 durch den Austragskolben 12 auf, die ringförmig um den zentralen Durchgang für den Mischstab 7 herum in dem Austragskolben 12 angeordnet sind und die Vorderseite des Austragskolbens 12 mit der Rückseite des Austragskolbens 12 und dadurch die beiden Seiten des Innenraums 11 der Kartusche 1 miteinander verbinden. Die Kanäle 14 sind mit einem ringförmigen Porenfilter 16 abgedeckt. Der Porenfilter 16 ist für das Knochenzementpulver 4 aus dem Innenraum 11 der Kartusche 1 undurchlässig und für die Monomerflüssigkeit 3 und Gase durchlässig. Dadurch wird ein Vordringen des Knochenzementpulvers 4 in das Innere der Aufnahme 2 verhindert.

Der Austragskolben 12 weist einen größeren Außendurchmesser auf, als das Außengewinde 9 der Aufnahme 2. Der Außendurchmesser des zylindrischen Austragskolbens 12 passt zum Innendurchmesser des Innenraums 11 der Kartusche 1. Der Austragskolben 12 dichtet den Innenraum 11 der Kartusche 1 ab.

An der Rückseite der Aufnahme 2 ist eine Öffnungseinrichtung 18 vorgesehen, mit der die Ampulle 5 in Richtung des Austragskolbens 12 gedrückt werden kann, um die Ampulle 5 im Inneren der Aufnahme 2 zu öffnen, so dass die Monomerflüssigkeit 3 in der Aufnahme 2 ausfließt. Die Öffnungseinrichtung 18 weist hierzu eine zweistufige Hülse 20 auf, wobei die Vorderseite der Hülse 20 einen Hohlzylinder bildet, in dem ein Ampullenkopf der Ampulle 5 angeordnet ist. Die Hülse 20 der Öffnungseinrichtung 18 kann dadurch auf Schultern 21 der Ampulle 5 drücken, um diese nach vorne in Richtung des Austragskolbens 12 zu drücken und dadurch zu öffnen. Da die Hülse 20 auf die Schultern 21 drückt, wird die Kraft durch den Ampullenkörper bis zu einem Ampullenboden 27 der Ampulle 5 geleitet. Die Wandungen des Ampullenkörpers sind sehr stabil, so dass die Ampulle 5 in diesem Bereich nicht brechen wird. Die Ampulle 5 kann so am Ampullenboden 27 aufgebrochen werden.

Die Hülse 20 liegt dabei an der Innenwand der Aufnahme 2 an und deckt diese im Bereich der Rückseite des Inneren der Aufnahme 2 ab. Das rückseitige Ende der Aufnahme 2 ist mit einer Verschlusskappe 22 der Öffnungseinrichtung 18 abgedeckt. In der Hülse 20 ist eine Wand senkrecht zur Achse der Zylindergeometrie der Hülse 20 vorgesehen, wobei in der Wand eine Öffnung 23 vorgesehen ist. Die Öffnung 23 verhindert, dass sich beim Einschieben der Aufnahme 2 in die Kartusche 1 eine Gasfederung bildet. Zudem kann die Monomerflüssigkeit 3 leichter aus der Aufnahme 2 fließen, wenn Luft durch die Öffnung 23 nachströmen kann. Der Knochenzementapplikator muss für die Anwendung mit dem Kartuschendeckel 6 nach unten gehalten oder aufgestellt werden, so wie das in den Figuren 1 bis 6 gezeigt ist. Die Hülse 20 ist an der schraubbaren Verschlusskappe 22 befestigt. Die Verschlusskappe 22 weist ein Innengewinde 24 auf, das zu dem Außengewinde 9 der Aufnahme 2 passt.

Die Verschlusskappe 22 beziehungsweise die Öffnungseinrichtung 18 ist ein kleines Stück aber nicht bis zu einem Anschlag auf die Rückseite der Aufnahme 2 aufgeschraubt und so an dieser befestigt. Es ist wichtig, dass die Verschlusskappe 22 noch weiter auf die Aufnahme 2 aufgeschraubt werden kann und dadurch die Hülse 20 tiefer in die Aufnahme 2 eingeschoben werden kann, damit die Ampulle 5 in der Aufnahme 2 geöffnet werden kann.

An der Rückseite der Aufnahme 2 ist eine umlaufende Nut in dem Gewinde 9 vorgesehen, in die ein Sicherungselement 26 in Form einer Spange aufgesteckt ist. Das Sicherungselement 26 verhindert ein ungewolltes Aufschrauben der Verschlusskappe 22 auf die Aufnahme 2 und damit ein ungewolltes Bedienen der Öffnungseinrichtung 18. Das Sicherungselement 26 kann unmittelbar vor einer Anwendung des Knochenzementapplikators gelöst werden, indem das Sicherungselement 26 abgezogen wird. Danach kann die Öffnungseinrichtung 18 in die Aufnahme 2 geschraubt werden.

Um eine Drehung der Verschlusskappe 22 in die falsche Richtung und dadurch ein Öffnen der Aufnahme 2 an deren Rückseite zu verhindern, ist eine Rückdrehsicherung vorgesehen (in den Figuren 1 bis 6 nicht zu sehen). Die Rückdrehsicherung verhindert ein Lösen der Verschlusskappe 22 beziehungsweise ein Lösen der Öffnungseinrichtung 18 von der Aufnahme 2. Die Rückdrehsicherung kann beispielsweise als Schraubensicherung in Form einer Sperrkantscheibe oder durch ein Keilsicherungsscheibenpaar oder ähnliche Maßnahmen realisiert werden.

Um die Öffnungseinrichtung 18 und die Aufnahme 2 bequem mit der Hand drehen zu können und um die Aufnahme 2 bequem in die Kartusche 1 einschieben und herausziehen zu können, ist deren rückseitiges Ende mit einem Griff 28 ausgestattet. Um die Hülse 20 gegen die Innenwand der Aufnahme 2 abzudichten, sind zwei umlaufende Dichtungen 30 aus Gummi in umlaufenden Nuten am vordersten Außenumfang der Hülse 20 angeordnet. Die Hülse 20 ist außen abgestuft und bildet so einen Anschlag, zusätzlich zu dem Anschlag, der durch die Verschlusskappe 22 gebildet wird, wobei die Anschläge ein weiteres Aufschrauben der Öffnungseinrichtung 18 auf beziehungsweise in die Aufnahme 2 verhindern.

In gleicher Weise befinden sich am Außenumfang des Austragskolbens 12 zwei in Längsrichtung voneinander beabstandete Nuten, in denen sich zwei umlaufende Dichtungen 32 aus Gummi befinden. Die Dichtungen 32 dichten den Austragskolben 12 gegen den Innenraum 11 der Kartusche 1 ab und trennen den Innenraum 11 der Kartusche 1 in einen vorderen Teil, in dem das Knochenzementpulver 4 angeordnet ist, und einen hinteren Teil.

An der zum Kartuschendeckel 6 weisenden vorderen Wandung der Aufnahme 2 ist ein Dorn 34 zum Aufbrechen der Ampulle 5 angeordnet. Der Dorn 34 weist hierzu ins Innere der Aufnahme 2. Die Ampulle 5 kann zum Öffnen der Ampulle 5 mit der Hülse 20 auf den Dorn 34 gedrückt werden, bis der Ampullenboden 27 der Ampulle 5 in den Ampullenkörper gedrückt wird. Der Dorn 34 hat eine stumpfe Spitze, die dazu dient, dass die Kraft auf die Ampulle 5 auf einen mittleren Bereich des Ampullenbodens 27 wirkt, so dass eine Sollbruchstelle in der Verbindung zwischen dem Ampullenboden 27 und den seitlichen Wandungen des Ampullenkörpers genutzt wird. Die Kraft hierzu wird über die Hülse 20 ausgeübt. Die Hülse 20 weist in etwa den gleichen Durchmesser wie der Ampullenkörper der Ampulle 5 auf. Der Ampullenkopf der Ampulle 5 ist dabei im Inneren der Hülse 20 angeordnet. Damit wird erreicht, dass die Ampulle 5 nicht im Bereich der Hülse 20 gebrochen wird, weil der zylindrische Ampullenkörper sehr stabil ist, während der Dorn 34 relativ leicht von vorne in die Ampulle 5 gedrückt werden kann.

Der Mischstab 7 ist innerhalb des Dorns 34 an der Aufnahme 2 befestigt. Der Dorn 34 ist über eine Sollbruchstelle mit der Aufnahme 2 verbunden, so dass bei einem Druck auf den Mischstab 7, der Mischstab 7 den Dorn 34 von der Aufnahme 2 abtrennt und so der Mischstab 7 mit dem Dorn 34 an der Spitze durch die vordere Grundfläche der Aufnahme 2 beweglich ist. Alternativ kann auch eine innere Kreisscheibe (nicht gezeigt) der Vorderseite der Aufnahme 2 über ein Gewinde mit der Aufnahme 2 verbunden sein, so dass der Dorn 34 mit dieser Kreisscheibe durch eine Drehung der Aufnahme 2 gegen den hierzu gegen den Kartuschendeckel 6 fixierten Mischstab 7 von der restlichen Aufnahme 2 getrennt werden kann, so dass der Mischstab 7 wieder gegen die restliche Aufnahme 2 beweglich ist.

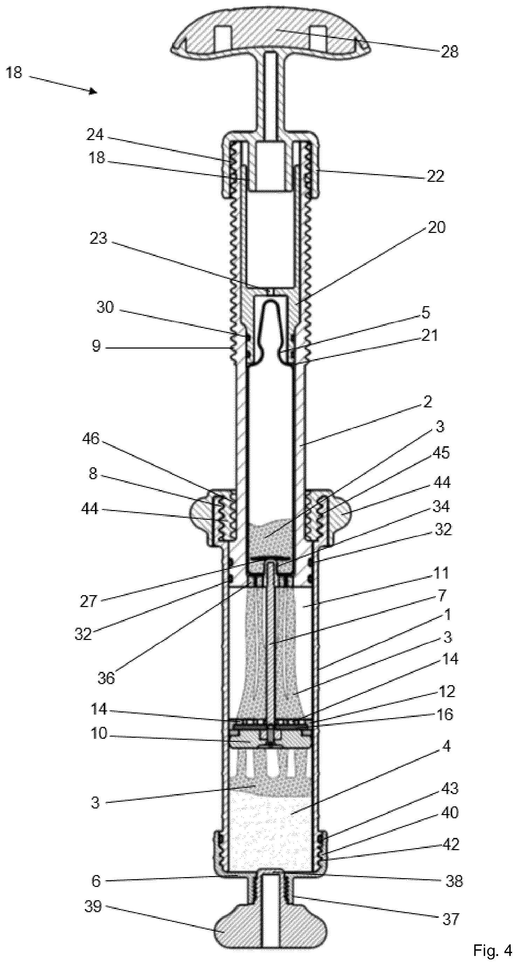

Um den Dorn 34 herum sind mehrere Durchgänge 36 angeordnet, die das Innere der Aufnahme 1 mit dem Innenraum 11 der Kartusche 1 verbinden. Durch die Durchgänge 36 kann die Monomerflüssigkeit 3 in den Innenraum 11 der Kartusche 1 fließen, so wie das in Figur 4 gezeigt ist.

Die Vorderseite der Kartusche 1 ist mit dem Kartuschendeckel 6 verschlossen. In der Mitte des Kartuschendeckels 6 ist ein Stutzen 37 ausgeformt, der die Austragsöffnung im Kartuschendeckel 6 begrenzt. In dem Stutzen 37 ist ein Verschluss 38 eingeschraubt und dadurch lösbar befestigt, der die Austragsöffnung verschließt. Der Verschluss 38 kann über Flügel 39 nach Art einer Flügelschraube bedient werden. Der Kartuschendeckel 6 ist mit einem Innengewinde 40 auf ein Außengewinde 42 an der Vorderseite der Kartusche 1 geschraubt. Der Kartuschendeckel 6 ist zusätzlich über eine umlaufende Dichtung 43 gegen die Kartusche 1 abgedichtet.

In dem vorderen Teil des Innenraums 11 der Kartusche 1 befindet sich der Mischer 10, mit dem der Inhalt des vorderen Teils des Innenraums 11 durch eine manuelle Bewegung des Mischers 10 durchmischt werden kann. Die manuelle Bewegung des Mischers 10 erfolgt durch Einschieben und Herausziehen der Aufnahme 2 in die Kartusche 1. Dabei wird nämlich auch der Mischstab 7, der an der Vorderseite der Aufnahme 2 befestigt ist, linear hin- und herbewegt. Der Mischstab 7 bewegt sich dabei durch die Durchführung im Austragskolben 12 und der an dem Mischstab 7 befestigte Mischer 10 bewegt sich in dem vorderen Teil des Innenraums 11 der Kartusche 1.

Das Innengewinde 8 an der Rückseite der Kartusche 1 hat einen größeren Durchmesser als der Innenraum 11. In das Innengewinde 8 ist ein Schraubring 44 mit einem zum Innengewinde 8 passenden Außengewinde 45 geschraubt. Der Schraubring 44 hat an der Innenseite ein Innengewinde 46, das zu dem Außengewinde 9 der Aufnahme 2 passt. Die Aufnahme 2 kann also in das Innengewinde 46 des Schraubrings 44 geschraubt werden (siehe Figur 6).

Zusätzlich dient das Innengewinde 46 des Schraubrings 44 als Anschlag für das Hineinschieben der Aufnahme 2 in die Kartusche 1. Wenn die Aufnahme 2 bis zum Anschlag in die Kartusche 1 eingeschoben wird, trifft nämlich das Außengewinde 9 der Aufnahme 2 auf das Innengewinde 46 des Schraubrings 44, der vollständig in das Innengewinde 8 an der Rückseite der Kartusche 1 eingeschraubt ist. Gleichzeitig ist die Länge des Mischstabs 7 genau so gewählt, dass der Mischer 10 an der Vorderseite des Innenraums 11 am Kartuschendeckel 6 anliegt. Dadurch kann der Knochenzement 48 auch an der Vorderseite des Innenraums 11 vollständig mit dem Mischer 10 erreicht und gemischt werden.

Der Verschluss 38 ragt ein kleines Stück in den Innenraum 11 der Kartusche 1 hinein. An der dem Kartuschendeckel 6 zugewandten Vorderseite des Mischers 10 ist eine Vertiefung vorgesehen, die den in den Innenraum 11 ragenden Teil des Verschlusses 38 aufnimmt. Dadurch kann auch der am Verschluss 38 und am Kartuschendeckel 6 anliegende Knochenzement 48 gemischt werden und gleichzeitig wird durch diese Vertiefung ein freier Strömungsquerschnitt für den Knochenzement 48 bereitgestellt, wenn der Verschluss 38 entfernt ist und der Mischer 10 beim Austragen des Knochenzements 48 am Kartuschendeckel 6 anliegt (siehe Figur 6).

Im Folgenden wird der Ablauf eines erfindungsgemäßen Verfahrens anhand der Figuren 1 bis 6 erläutert. Zunächst befindet sich der Knochenzementapplikator im Ausgangszustand (siehe Figuren 1 bis 3). In diesem Zustand ist der Knochenzementapplikator verpackt und mit Ethylenoxid sterilisiert worden. Das Ethylenoxid kann durch Zwischenräume in der Öffnungseinrichtung 18 und durch die Öffnung 23 in das Innere der Aufnahme 2 und durch die Durchgänge 36, den Porenfilter 16 und die Kanäle 14 in den Innenraum 11 der Kartusche 1 gelangen. Der Gasaustausch erfolgt dabei in einer Vakuumkammer oder Unterdruckkammer. In diesem Zustand (siehe Figuren 1 bis 3) wird der Knochenzementapplikator ausgepackt.

Zunächst wird das Sicherungselement 26 abgezogen. Der Knochenzementapplikator wird mit dem Kartuschendeckel 6 nach unten gehalten. Anschließend wird die Öffnungseinrichtung 18 in die Aufnahme 2 geschraubt. Auch hierbei wird der Knochenzementapplikator mit dem Kartuschendeckel 6 nach unten gehalten. Dabei drückt die Hülse 20 die Ampulle 5 an deren Schultern 21 in Richtung nach unten. Anschließend wird die Ampulle 5 mit dem Ampullenboden 27 auf den Dorn 34 gedrückt und die Ampulle 5 bricht am Ampullenboden 27 auf. Dieser Zustand ist in Figur 4 gezeigt.

Die Monomerflüssigkeit 3 tritt aus der geöffneten Ampulle 5 im Bereich der Durchgänge 36 aus. Da der Knochenzementapplikator mit dem Kartuschendeckel 6 nach unten gehalten wird, fließt die Monomerflüssigkeit 3 getrieben von der Schwerkraft sofort nach unten durch die Durchgänge 36, den Porenfilter 16 und die Kanäle 14 in den Innenraum 11 der Kartusche 1 und verteilt sich in dem Knochenzementpulver 4 (siehe Figur 4). Um den Monomertransfer zu beschleunigen kann die Aufnahme 2 in die Kartusche 1 hineingeschoben und herausgezogen werden.

Das Mischen des Knochenzements 48 beziehungsweise der Ausgangskomponenten 3, 4 des Knochenzements 48 erfolgt durch Einschieben und Herausziehen der Aufnahme 2 aus der Kartusche 1 bei gleichzeitiger Bewegung des Mischers 10 im Innenraum 11 der Kartusche 1. Der Mischer 10 erreicht dabei alle Orte in dem Innenraum 11 zwischen dem Austragskolben 12 und dem Kartuschendeckel 6. Zum Führen dieser Bewegung sind außen an der Aufnahme 2 in dem vorderen Bereich ohne das Außengewinde 9 Leisten 47 angeordnet, die an dem Innengewinde 46 des Schraubrings 44 anliegen. Die Leisten 47 verhindern, dass die Aufnahme 2 beim Mischen wackelt. In den Zwischenräumen kann Luft entweichen, die ansonsten zwischen dem Schraubring 44, der Außenwand der Aufnahme 2, der Innenwand der Kartusche 1 und der Dichtung 32 eingeschlossen wäre. Hiermit wird verhindert, dass beim Mischen des Knochenzements 48 beziehungsweise der Ausgangskomponenten 3, 4 gegen die Kraft einer Gasfederung in diesem Bereich gearbeitet werden muss.

Schließlich ist der Knochenzement 48 gemischt und die Aufnahme 2 wird vollständig in die Kartusche 1 eingeschoben, so dass der Mischer 10 an dem Kartuschendeckel 6 anliegt. Diese Situation ist in Figur 5 dargestellt.

Um die Aufnahme 2 in das Innengewinde 46 des Schraubrings 44 schrauben zu können, muss der Schraubring 44 nach dem Mischen des Knochenzements 48 ein Stück aus dem Innengewinde 8 der Kartusche 1 herausgeschraubt werden. Dadurch lässt sich das Innengewinde 46 des Schraubrings 44 mit dem Außengewinde 9 der Aufnahme 2 in Eingriff bringen. Der Schraubring 44 darf dabei nicht vollständig von der Kartusche 1 gelöst werden. Hierzu kann ein geeignetes Verbindungsmittel (nicht gezeigt) vorgesehen sein. Auf diese Weise kann die Aufnahme 2 in den Schraubring 44 und damit in die Kartusche 1 hinein geschraubt werden. Dadurch lässt sich die Aufnahme 2 kraftvoll in den die Kartusche 1 drücken. Der Mischer 10 liegt vorne am Kartuschendeckel 6 an, so dass der Mischstab 7 nicht ausweichen kann. Der von dem Mischstab 7 vermittelte Druck löst den Dorn 34 von der vorderen Wandung der Aufnahme 2 oder der Mischstab 7 durchstößt den Dorn 34. Gleichzeitig wird auch der in Presspassung gehaltene Austragskolben 12 von der Aufnahme 2 gelöst und in Richtung des Kartuschendeckels 6 getrieben.

Bei weiterem Einschrauben der Aufnahme 2 in die Kartusche 1 wird der Knochenzement 48 aus dem Innenraum 11 der Kartusche 1 durch die geöffnete Austragsöffnung ausgetrieben. Hierzu wird der Verschluss 38 zuvor aus der Austragsöffnung herausgeschraubt und ein Austragsrohr 49 wird in das Innengewinde des Stutzens 37 geschraubt. Das Austragsrohr 49 hat dafür ein zum Innengwinde des Stutzens 37 passendes Außengewinde. Der Knochenzement 48 wird zwischen dem Mischer 10 und dem Kartuschendeckel 6 durch die Austragsöffnung und den Stutzen 37 in das Austragsrohr 49 gepresst. Anschließend fließt der Knochenzement 48 aus dem Austragsrohr 49 hinaus und ist zur Anwendung bereit (siehe Figur 6).

Während des Auspressens des Knochenzements 48 werden Gaseinschlüsse in dem Knochenzement 48 durch den Porenfilter 16 nach oben in die Aufnahme 2 gedrückt, so dass ein blasenarmer Knochenzement 48 erzeugt wird.

Alternativ zu dem Austragsrohr 49 kann auch ein Schlauch mit einem Trokar (nicht gezeigt) an dem Stutzen 37 befestigt werden, durch die der Knochenzement 48 an schwer zugänglichen Orten unter Röntgenkontrolle appliziert werden kann.

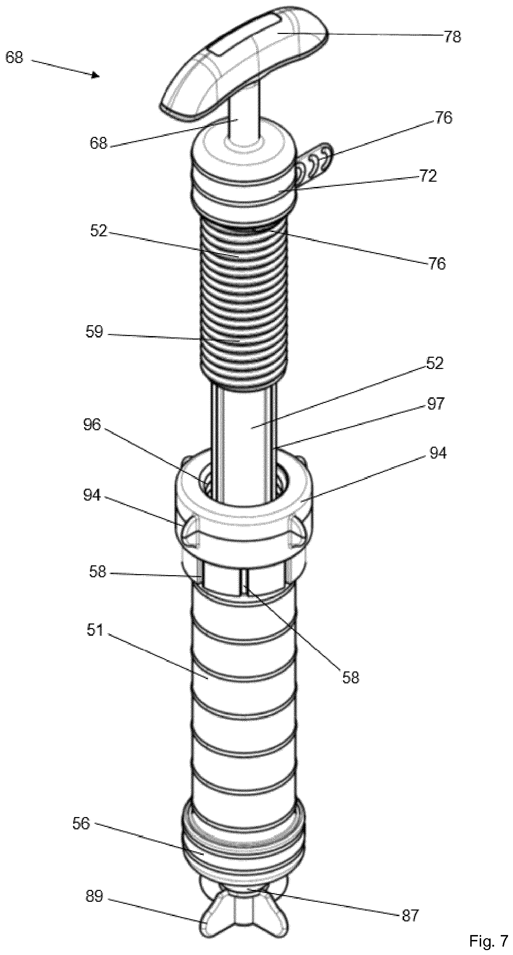

In den Figuren 7 bis 12 sind Abbildungen eines zweiten alternativen erfindungsgemäßen Knochenzementapplikators zum Lagern von Ausgangskomponenten 3, 4 eines Knochenzements 48 und zum Mischen des Knochenzements 48 gezeigt. Die Figuren zeigen dabei auch den Ablauf eines erfindungsgemäßen Verfahrens durchgeführt mit dem zweiten erfindungsgemäßen Knochenzementapplikator in Form von fünf Querschnittansichten des zweiten erfindungsgemäßen Knochenzementapplikators.

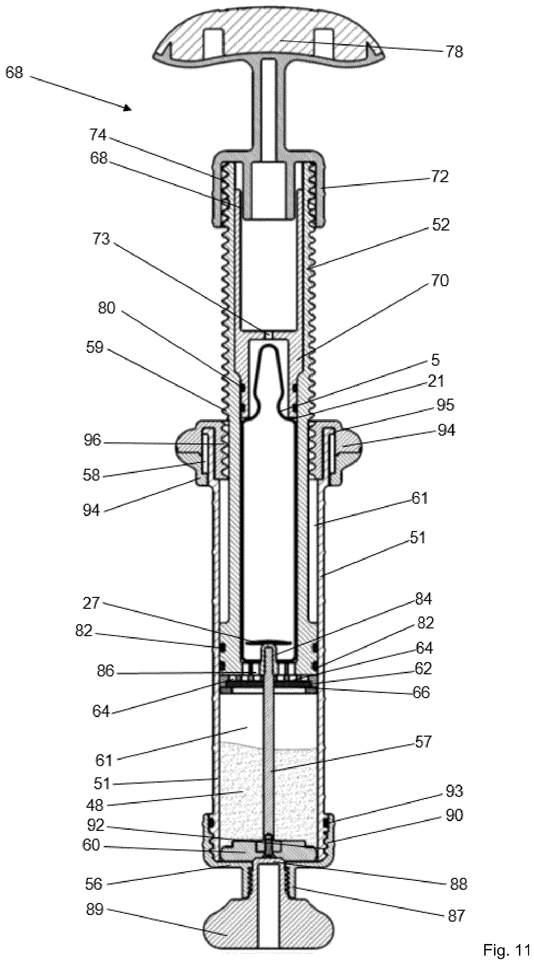

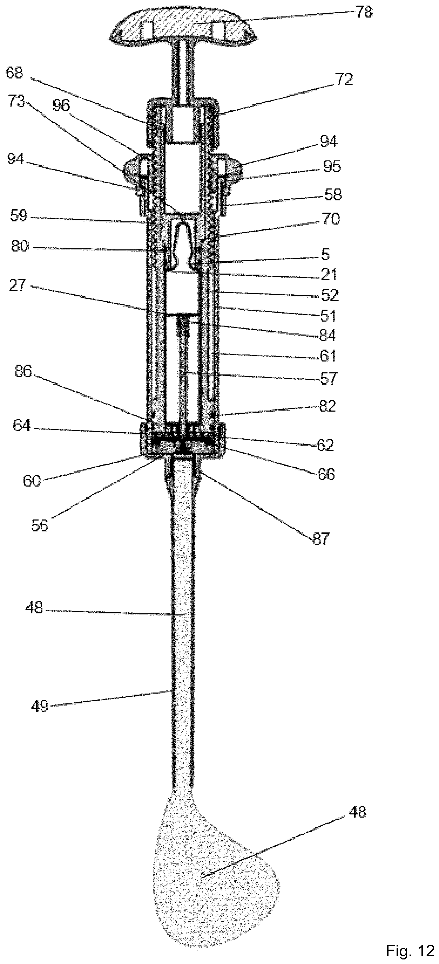

Der zweite erfindungsgemäße Knochenzementapplikator entspricht in seinem Aufbau weitgehend dem ersten erfindungsgemäßen Knochenzementapplikator nach den Figuren 1 bis 6. Der zweite erfindungsgemäße Knochenzementapplikator weist eine röhrenförmige Kartusche 51 aus Kunststoff auf, die einen vorderen Teil (in den Figuren 7 bis 12 unten) des Knochenzementapplikators bildet. Ein rückseitiger, hinterer Teil des Knochenzementapplikators wird durch eine Aufnahme 52 gebildet. Der Knochenzementapplikator ist zur Herstellung eines Knochenzements 48 (siehe Figuren 11 und 12) vorgesehen, der aus einer Monomerflüssigkeit 3 und aus einem Knochenzementpulver 4 hergestellt wird. Die Ausgangskomponenten 3, 4 sind also die gleichen, die auch bei dem ersten Ausführungsbeispiel zu den Figuren 1 bis 6 verwendet werden. Die Monomerflüssigkeit 3 ist auch in einer gleichen aufbrechbaren Ampulle 5 aus Glas oder aus einem Kunststoff als Monomerflüssigkeitsbehälter für die Monomerflüssigkeit 3 enthalten, wobei die Ampulle 5 in der Aufnahme 52 steckt. Die Kartusche 51 bildet in ihrem Inneren einen zylindrischen Innenraum 61, in dem das Knochenzementpulver 4 enthalten ist. Somit ist der Knochenzementapplikator auch zum Lagern der Monomerflüssigkeit 3 und des Knochenzementpulvers 4 geeignet.

Die Kartusche 51 weist an ihrer Vorderseite (in den Figuren unten) einen Kartuschendeckel 56 als Kartuschenkopf auf. In dem Kartuschendeckel 56 ist eine Austragsöffnung vorgesehen. An der Vorderseite der Aufnahme 52 ist ein Mischstab 57 befestigt, der sich von der Vorderseite der Aufnahme 52 bis in den vorderen Teil der Kartusche 51 erstreckt, in dem sich das Knochenzementpulver 4 befindet.

Die Aufnahme 52 weist an ihrer Außenseite ein Außengewinde 59 auf. Die Aufnahme 52 ist in einem hinteren Bereich nach Art eines Gewinderohrs geformt und weist in ihrem Inneren eine zylindrische Kammer auf, in der die Ampulle 5 steckt. In einem vorderen Bereich ist die Aufnahme 52 außen zylindrisch geformt, wobei vier vorspringende Leisten 97 parallel zur Zylinderachse der Aufnahme 52 an der äußeren Oberfläche der Aufnahme 52 vorgesehen sind. Die Ampulle 5 hat einen zylindrischen Ampullenkörper mit einem zu dem Inneren der Aufnahme 2 passendem Durchmesser. Im Inneren der Kartusche 51 bildet die Kartusche 51 den zylindrischen Innenraum 61. Die Zylindergeometrie des Innenraums 61 und der Kammer der Aufnahme 52 entsprechen Zylindern mit kreisförmiger Grundfläche.

An der Vorderseite des Mischstabs 57 ist ein Mischer 60 in Form von Mischflügeln mit einem umgebenden Abkratzring befestigt. Mit dem Abkratzring können die Bereiche direkt an der Innenwand des Innenraums 61 erreicht werden.

Die Aufnahme 52 ist an ihrer Vorderseite durch eine Wandung mit mehreren Durchgängen 86 als Verschluss der Vorderseite begrenzt, wobei die Wandung an der Vorderseite der Aufnahme 52 die Kammer nach vorne an ihrer kreisförmigen Grundfläche verschließt. Im Innenraum 61 der Kartusche 51 ist ein in axialer Richtung des zylindrischen Innenraums 61 beweglicher Austragskolben 62 angeordnet, der in Presspassung in dem Innenraum 61 angeordnet ist. Der Mischstab 57 ist durch einen zentralen Durchgang im Austragskolben 62 geführt, so dass der Mischstab 57 gegen den Austragskolben 62 bewegt werden kann, ohne dass sich dabei der Austragskolben 62 im Innenraum der Kartusche 51 bewegt. Bei zurückgezogener Aufnahme 52 liegt der Mischer 60 an der Vorderseite des Austragskolbens 62 an. Dadurch kann der gesamte vordere Teil des Innenraums 61, der seitlich durch die Kartusche 51, vorne durch den Kartuschendeckel 56 und hinten durch den Austragskolben 62 begrenzt ist, von dem Mischer 60 erreicht werden. Dadurch ist eine vollständige Durchmischung des Knochenzementpulvers 4 mit der Monomerflüssigkeit 3 in diesem Bereich gewährleistet.

Der Austragskolben 62 weist mehrere Kanäle 64 durch den Austragskolben 62 auf, die ringförmig um den zentralen Durchgang für den Mischstab 57 herum in dem Austragskolben 62 angeordnet sind und die Vorderseite des Austragskolbens 62 mit der Rückseite des Austragskolbens 62 und dadurch die beiden Seiten des Innenraums 61 der Kartusche 51 miteinander verbinden. Die Kanäle 64 sind mit einem ringförmigen Porenfilter 66 abgedeckt. Der Porenfilter 66 ist für das Knochenzementpulver 4 aus dem Innenraum 61 der Kartusche 51 undurchlässig und für die Monomerflüssigkeit 3 und Gase durchlässig. Dadurch wird ein Vordringen des Knochenzementpulvers 4 in das Innere der Aufnahme 52 verhindert.

Der Austragskolben 62 weist einen größeren Außendurchmesser auf, als das Außengewinde 59 der Aufnahme 52. Der Außendurchmesser des zylindrischen Austragskolbens 62 passt zum Innendurchmesser des Innenraums 61 der Kartusche 51. Der Austragskolben 62 dichtet den Innenraum 61 der Kartusche 51 ab.

An der Rückseite der Aufnahme 52 ist eine Öffnungseinrichtung 68 vorgesehen, mit der die Ampulle 5 in Richtung des Austragskolbens 62 gedrückt werden kann, um die Ampulle 5 im Inneren der Aufnahme 52 zu öffnen, so dass die Monomerflüssigkeit 3 in der Aufnahme 52 ausfließt. Die Öffnungseinrichtung 68 weist hierzu eine zweistufige Hülse 70 auf, wobei die Vorderseite der Hülse 70 einen Hohlzylinder bildet, in dem ein Ampullenkopf der Ampulle 5 angeordnet ist. Die Hülse 70 der Öffnungseinrichtung 68 kann dadurch auf Schultern 21 der Ampulle 5 drücken, um diese nach vorne in Richtung des Austragskolbens 62 zu drücken und dadurch zu öffnen. Da die Hülse 70 auf die Schultern 21 drückt, wird die Kraft durch den Ampullenkörper bis zu einem Ampullenboden 27 der Ampulle 5 geleitet. Die Wandungen des Ampullenkörpers sind sehr stabil, so dass die Ampulle 5 in diesem Bereich nicht brechen wird. Die Ampulle 5 kann so am Ampullenboden 27 aufgebrochen werden.

Die Hülse 70 liegt dabei an der Innenwand der Aufnahme 52 an und deckt diese im Bereich der Rückseite des Inneren der Aufnahme 52 ab. Das rückseitige Ende der Aufnahme 52 ist mit einer Verschlusskappe 72 der Öffnungseinrichtung 68 abgedeckt. In der Hülse 70 ist eine Wand senkrecht zur Achse der Zylindergeometrie der Hülse 70 vorgesehen, wobei in der Wand eine Öffnung 73 vorgesehen ist. Die Öffnung 73 verhindert, dass sich beim Einschieben der Aufnahme 52 in die Kartusche 51 eine Gasfederung bildet. Zudem kann die Monomerflüssigkeit 3 leichter aus der Aufnahme 52 fließen, wenn Luft durch die Öffnung 73 nachströmen kann. Der Knochenzementapplikator muss für die Anwendung mit dem Kartuschendeckel 56 nach unten gehalten oder aufgestellt werden, so wie das in den Figuren 7 bis 12 gezeigt ist. Die Hülse 70 ist an der schraubbaren Verschlusskappe 72 befestigt. Die Verschlusskappe 72 weist ein Innengewinde 74 auf, das zu dem Außengewinde 59 der Aufnahme 52 passt.

Die Verschlusskappe 72 beziehungsweise die Öffnungseinrichtung 68 ist ein kleines Stück aber nicht bis zu einem Anschlag auf die Rückseite der Aufnahme 52 aufgeschraubt und so an dieser befestigt. Es ist wichtig, dass die Verschlusskappe 72 noch weiter auf die Aufnahme 52 aufgeschraubt werden kann und dadurch die Hülse 70 tiefer in die Aufnahme 52 eingeschoben werden kann, damit die Ampulle 5 in der Aufnahme 52 geöffnet werden kann.

An der Rückseite der Aufnahme 52 ist eine umlaufende Nut in dem Gewinde 59 vorgesehen, in die ein Sicherungselement 76 in Form einer Spange aufgesteckt ist. Das Sicherungselement 76 verhindert ein ungewolltes Aufschrauben der Verschlusskappe 72 auf die Aufnahme 52 und damit ein ungewolltes Bedienen der Öffnungseinrichtung 68. Das Sicherungselement 76 kann unmittelbar vor einer Anwendung des Knochenzementapplikators gelöst werden, indem das Sicherungselement 76 abgezogen wird. Danach kann die Öffnungseinrichtung 68 in die Aufnahme 52 geschraubt werden.

Um eine Drehung der Verschlusskappe 72 in die falsche Richtung und dadurch ein Öffnen der Aufnahme 52 an deren Rückseite zu verhindern, ist eine Rückdrehsicherung vorgesehen (in den Figuren 7 bis 12 nicht zu sehen). Die Rückdrehsicherung verhindert ein Lösen der Verschlusskappe 72 beziehungsweise ein Lösen der Öffnungseinrichtung 68 von der Aufnahme 52. Die Rückdrehsicherung kann beispielsweise als Schraubensicherung in Form einer Sperrkantscheibe oder durch ein Keilsicherungsscheibenpaar oder ähnliche Maßnahmen realisiert werden.

Um die Öffnungseinrichtung 68 und die Aufnahme 52 bequem mit der Hand drehen zu können und um die Aufnahme 52 bequem in die Kartusche 51 einschieben und herausziehen zu können, ist deren rückseitiges Ende mit einem Griff 78 ausgestattet. Um die Hülse 70 gegen die Innenwand der Aufnahme 52 abzudichten, sind zwei umlaufende Dichtungen 80 aus Gummi in umlaufenden Nuten am vordersten Außenumfang der Hülse 70 angeordnet. Die Hülse 70 ist außen abgestuft und bildet so einen Anschlag, zusätzlich zu dem Anschlag, der durch die Verschlusskappe 72 gebildet wird, wobei die Anschläge ein weiteres Aufschrauben der Öffnungseinrichtung 68 auf beziehungsweise in die Aufnahme 52 verhindern.

In gleicher Weise befinden sich am Außenumfang des Austragskolbens 62 zwei in Längsrichtung voneinander beabstandete Nuten, in denen sich zwei umlaufende Dichtungen 82 aus Gummi befinden. Die Dichtungen 82 dichten den Austragskolben 62 gegen den Innenraum 61 der Kartusche 51 ab und trennen den Innenraum 61 der Kartusche 51 in einen vorderen Teil, in dem das Knochenzementpulver 4 angeordnet ist, und einen hinteren Teil.

An der zum Kartuschendeckel 56 weisenden vorderen Wandung der Aufnahme 52 ist ein Dorn 84 zum Aufbrechen der Ampulle 5 angeordnet. Der Dorn 84 weist hierzu ins Innere der Aufnahme 2. Die Ampulle 5 kann zum Öffnen der Ampulle 5 mit der Hülse 70 auf den Dorn 84 gedrückt werden, bis der Ampullenboden 27 der Ampulle 5 in den Ampullenkörper gedrückt wird. Der Dorn 84 hat eine stumpfe Spitze, die dazu dient, dass die Kraft auf die Ampulle 5 auf einen mittleren Bereich des Ampullenbodens 27 wirkt, so dass eine Sollbruchstelle in der Verbindung zwischen dem Ampullenboden 27 und den seitlichen Wandungen des Ampullenkörpers genutzt wird. Die Kraft hierzu wird über die Hülse 70 ausgeübt. Die Hülse 70 weist in etwa den gleichen Durchmesser wie der Ampullenkörper der Ampulle 5 auf. Der Ampullenkopf der Ampulle 5 ist dabei im Inneren der Hülse 70 angeordnet. Damit wird erreicht, dass die Ampulle 5 nicht im Bereich der Hülse 70 gebrochen wird, weil der zylindrische Ampullenkörper sehr stabil ist, während der Dorn 84 relativ leicht von vorne in die Ampulle 5 gedrückt werden kann.

Der Mischstab 57 ist innerhalb des Dorns 84 an der Aufnahme 52 befestigt. Der Dorn 84 ist über eine Sollbruchstelle mit der Aufnahme 52 verbunden, so dass bei einem Druck auf den Mischstab 57, der Mischstab 57 den Dorn 84 von der Aufnahme 52 abtrennt und so der Mischstab 57 mit dem Dorn 84 an der Spitze durch die vordere Grundfläche der Aufnahme 52 beweglich ist. Alternativ kann auch eine innere Kreisscheibe (nicht gezeigt) der Vorderseite der Aufnahme 52 über ein Gewinde mit der Aufnahme 52 verbunden sein, so dass der Dorn 84 mit dieser Kreisscheibe durch eine Drehung der Aufnahme 52 gegen den hierzu gegen den Kartuschendeckel 56 fixierten Mischstab 57 von der restlichen Aufnahme 52 getrennt werden kann, so dass der Mischstab 57 wieder gegen die restliche Aufnahme 52 beweglich ist.

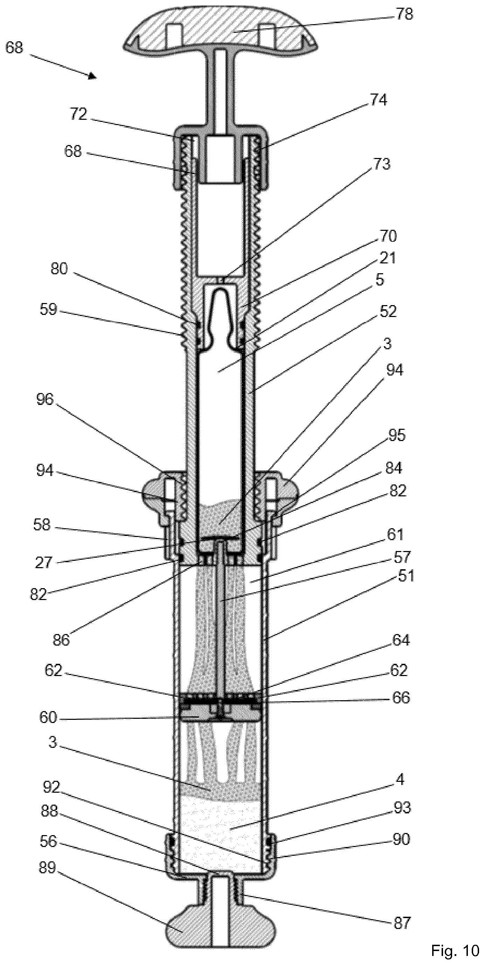

Um den Dorn 84 herum sind mehrere Durchgänge 86 angeordnet, die das Innere der Aufnahme 51 mit dem Innenraum 61 der Kartusche 51 verbinden. Durch die Durchgänge 86 kann die Monomerflüssigkeit 3 in den Innenraum 61 der Kartusche 51 fließen, so wie das in Figur 10 gezeigt ist.

Die Vorderseite der Kartusche 51 ist mit dem Kartuschendeckel 56 verschlossen. In der Mitte des Kartuschendeckels 56 ist ein Stutzen 87 ausgeformt, der die Austragsöffnung im Kartuschendeckel 56 begrenzt. In dem Stutzen 87 ist ein Verschluss 88 eingeschraubt und dadurch lösbar befestigt, der die Austragsöffnung verschließt. Der Verschluss 88 kann über Flügel 89 nach Art einer Flügelschraube bedient werden. Der Kartuschendeckel 56 ist mit einem Innengewinde 90 auf ein Außengewinde 92 an der Vorderseite der Kartusche 51 geschraubt. Der Kartuschendeckel 56 ist zusätzlich über eine umlaufende Dichtung 93 gegen die Kartusche 51 abgedichtet.

In dem vorderen Teil des Innenraums 61 der Kartusche 51 befindet sich der Mischer 60, mit dem der Inhalt des vorderen Teils des Innenraums 61 durch eine manuelle Bewegung des Mischers 60 durchmischt werden kann. Die manuelle Bewegung des Mischers 60 erfolgt durch Einschieben und Herausziehen der Aufnahme 52 in die Kartusche 51. Dabei wird nämlich auch der Mischstab 57, der an der Vorderseite der Aufnahme 52 befestigt ist, linear hin- und herbewegt. Der Mischstab 57 bewegt sich dabei durch die Durchführung im Austragskolben 62 und der an dem Mischstab 57 befestigte Mischer 60 bewegt sich in dem vorderen Teil des Innenraums 61 der Kartusche 51.

An der Rückseite der Kartusche 51 ist ein zweiteiliger Schiebering 94 befestigt. Ein Anschlag 95 an der Rückseite der Kartusche 51 verhindert, dass der Schiebering 94 von der Kartusche 51 getrennt werden kann. Die zwei Teile des Schieberings 94 werden fest miteinander verbunden, um den Schiebering 94 auf den Anschlag 95 einbauen zu können. In der Außenwand der Kartusche 51 sind im Bereich des Schieberings 94 Nuten 58 vorgesehen, in die Vorsprünge des Schieberings 94 greifen, so dass der Schiebering 94 nur entlang der Nuten 58 linear beweglich ist. Der Schiebering 94 hat an der Innenseite ein Innengewinde 96, das zu dem Außengewinde 59 der Aufnahme 52 passt. Die Aufnahme 52 kann also in das Innengewinde 96 des Schieberings 94 geschraubt werden (siehe Figur 12).

Zusätzlich dient das Innengewinde 96 des Schieberings 94 als Anschlag für das Hineinschieben der Aufnahme 52 in die Kartusche 51. Wenn die Aufnahme 52 bis zum Anschlag in die Kartusche 51 eingeschoben wird, trifft nämlich das Außengewinde 59 der Aufnahme 52 auf das Innengewinde 96 des Schieberings 94, der dabei vollständig in Richtung des Kartuschendeckels 56 geschoben wird. Gleichzeitig ist die Länge des Mischstabs 57 genau so gewählt, dass der Mischer 60 an der Vorderseite des Innenraums 61 am Kartuschendeckel 56 anliegt. Dadurch kann der Knochenzement 48 auch an der Vorderseite des Innenraums 61 vollständig mit dem Mischer 60 erreicht und gemischt werden.

Der Verschluss 88 ragt ein kleines Stück in den Innenraum 61 der Kartusche 51 hinein. An der dem Kartuschendeckel 56 zugewandten Vorderseite des Mischers 60 ist eine Vertiefung vorgesehen, die den in den Innenraum 61 ragenden Teil des Verschlusses 88 aufnimmt. Dadurch kann auch der am Verschluss 88 und am Kartuschendeckel 56 anliegende Knochenzement 48 gemischt werden und gleichzeitig wird durch diese Vertiefung ein freier Strömungsquerschnitt für den Knochenzement 48 bereitgestellt, wenn der Verschluss 88 entfernt ist und der Mischer 60 beim Austragen des Knochenzements 48 am Kartuschendeckel 56 anliegt (siehe Figur 12).

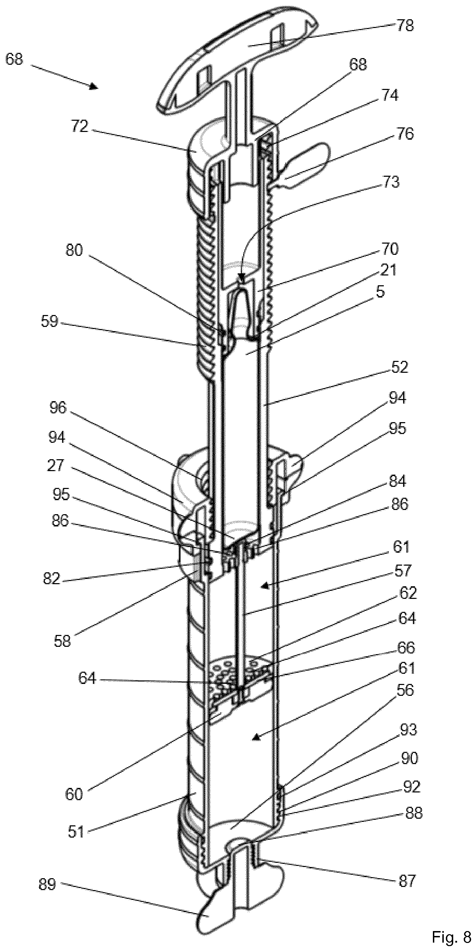

Im Folgenden wird der Ablauf eines erfindungsgemäßen Verfahrens anhand der Figuren 7 bis 12 erläutert. Zunächst befindet sich der Knochenzementapplikator im Ausgangszustand (siehe Figuren 7 und 8). In diesem Zustand ist der Knochenzementapplikator verpackt und mit Ethylenoxid sterilisiert worden. Das Ethylenoxid kann durch Zwischenräume in der Öffnungseinrichtung 68 und durch die Öffnung 73 in das Innere der Aufnahme 52 und durch die Durchgänge 86, den Porenfilter 66 und die Kanäle 64 in den Innenraum 61 der Kartusche 51 gelangen. Der Gasaustausch erfolgt dabei in einer Vakuumkammer oder Unterdruckkammer. In diesem Zustand (siehe Figuren 7 und 8) wird der Knochenzementapplikator ausgepackt.

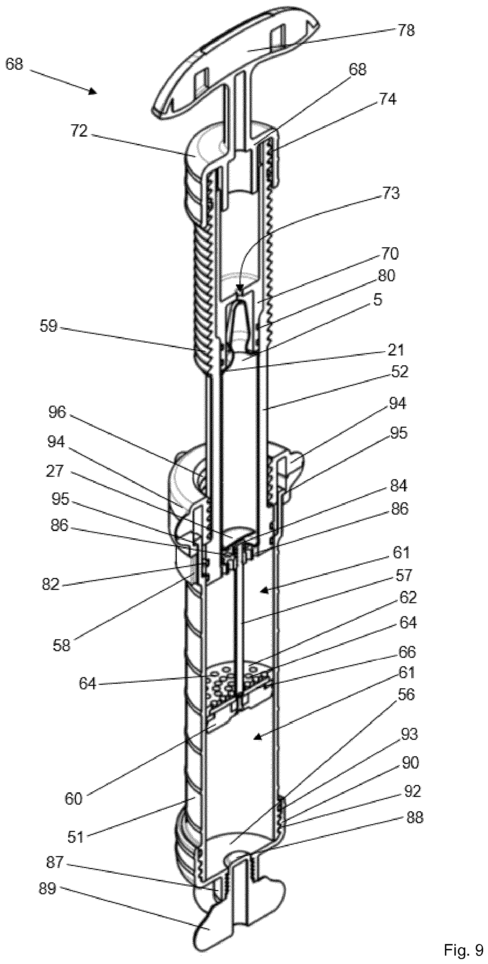

Zunächst wird das Sicherungselement 76 abgezogen. Der Knochenzementapplikator wird mit dem Kartuschendeckel 56 nach unten gehalten. Anschließend wird die Öffnungseinrichtung 68 in die Aufnahme 52 geschraubt. Auch hierbei wird der Knochenzementapplikator mit dem Kartuschendeckel 56 nach unten gehalten. Dabei drückt die Hülse 70 die Ampulle 5 an deren Schultern 21 in Richtung nach unten. Anschließend wird die Ampulle 5 mit dem Ampullenboden 27 auf den Dorn 84 gedrückt und die Ampulle 5 bricht am Ampullenboden 27 auf. Dieser Zustand ist in den Figuren 9 und 10 gezeigt.

Die Monomerflüssigkeit 3 tritt aus der geöffneten Ampulle 5 im Bereich der Durchgänge 86 aus. Da der Knochenzementapplikator mit dem Kartuschendeckel 56 nach unten gehalten wird, fließt die Monomerflüssigkeit 3 getrieben von der Schwerkraft sofort nach unten durch die Durchgänge 86, den Porenfilter 66 und die Kanäle 64 in den Innenraum 61 der Kartusche 51 und verteilt sich in dem Knochenzementpulver 4 (siehe Figur 10). Um den Monomertransfer zu beschleunigen kann die Aufnahme 52 in die Kartusche 51 hineingeschoben und herausgezogen werden.

Das Mischen des Knochenzements 48 beziehungsweise der Ausgangskomponenten 3, 4 des Knochenzements 48 erfolgt durch Einschieben und Herausziehen der Aufnahme 52 aus der Kartusche 51 bei gleichzeitiger Bewegung des Mischers 60 im Innenraum 61 der Kartusche 51. Der Mischer 60 erreicht dabei alle Orte in dem Innenraum 61 zwischen dem Austragskolben 62 und dem Kartuschendeckel 56. Zum Führen dieser Bewegung sind außen an der Aufnahme 52 in dem vorderen Bereich ohne das Außengewinde 59 Leisten 97 angeordnet, die an dem Innengewinde 96 des Schieberings 94 anliegen. Die Leisten 97 verhindern, dass die Aufnahme 52 beim Mischen wackelt. In den Zwischenräumen kann Luft entweichen, die ansonsten zwischen dem Schiebering 94, der Außenwand der Aufnahme 52, der Innenwand der Kartusche 51 und der Dichtung 82 eingeschlossen wäre. Hiermit wird verhindert, dass beim Mischen des Knochenzements 48 beziehungsweise der Ausgangskomponenten 3, 4 gegen die Kraft einer Gasfederung in diesem Bereich gearbeitet werden muss.

Schließlich ist der Knochenzement 48 gemischt und die Aufnahme 52 wird vollständig in die Kartusche 51 eingeschoben, so dass der Mischer 60 an dem Kartuschendeckel 56 anliegt. Diese Situation ist in Figur 11 dargestellt.

Um die Aufnahme 52 in das Innengewinde 96 des Schieberings 94 schrauben zu können, wird der Schiebering 94 nach dem Mischen des Knochenzements 48 bis zum Anschlag 95 aus der Kartusche 1 herausgezogen. Dadurch lässt sich das Innengewinde 96 des Schieberings 94 mit dem Außengewinde 59 der Aufnahme 52 in Eingriff bringen. Der Schiebering 94 kann aufgrund des Anschlags 95 nicht vollständig von der Kartusche 51 gelöst werden. Auf diese Weise kann die Aufnahme 52 in den Schiebering 94 und damit in die Kartusche 51 hinein geschraubt werden. Dadurch lässt sich die Aufnahme 52 kraftvoll in den die Kartusche 51 drücken. Der Mischer 60 liegt vorne am Kartuschendeckel 56 an, so dass der Mischstab 57 nicht ausweichen kann. Der von dem Mischstab 57 vermittelte Druck löst den Dorn 84 von der vorderen Wandung der Aufnahme 52 oder der Mischstab 57 durchstößt den Dorn 84. Gleichzeitig wird auch der in Presspassung gehaltene Austragskolben 62 von der Aufnahme 52 gelöst und in Richtung des Kartuschendeckels 56 getrieben.

Bei weiterem Einschrauben der Aufnahme 52 in die Kartusche 51 wird der Knochenzement 48 aus dem Innenraum 61 der Kartusche 51 durch die geöffnete Austragsöffnung ausgetrieben. Hierzu wird der Verschluss 88 zuvor aus der Austragsöffnung herausgeschraubt und ein Austragsrohr 49 wird in das Innengewinde des Stutzens 87 geschraubt. Das Austragsrohr 49 hat dafür ein zum Innengwinde des Stutzens 87 passendes Außengewinde. Der Knochenzement 48 wird zwischen dem Mischer 60 und dem Kartuschendeckel 56 durch die Austragsöffnung und den Stutzen 87 in das Austragsrohr 49 gepresst. Anschließend fließt der Knochenzement 48 aus dem Austragsrohr 49 hinaus und ist zur Anwendung bereit (siehe Figur 12).

Während des Auspressens des Knochenzements 48 werden Gaseinschlüsse in dem Knochenzement 48 durch den Porenfilter 66 nach oben in die Aufnahme 52 gedrückt, so dass ein blasenarmer Knochenzement 48 erzeugt wird.

Alternativ zu dem Austragsrohr 49 kann auch ein Schlauch mit einem Trokar (nicht gezeigt) an dem Stutzen 87 befestigt werden, durch die der Knochenzement 48 an schwer zugänglichen Orten unter Röntgenkontrolle appliziert werden kann.

### Bezugszeichenliste

- 1, 51: Kartusche
- 2, 52: Aufnahme / Ampullenhalter
- 3: Monomerflüssigkeit
- 4: Knochenzementpulver
- 5: Ampulle
- 6, 56: Kartuschendeckel / Kartuschenkopf
- 7, 57: Mischstab
- 8: Innengewinde
- 9, 59: Außengewinde
- 10, 60: Mischer / Mischflügel
- 11, 61: Innenraum
- 12, 62: Austragskolben
- 14, 64: Kanal
- 16, 66: Porenfilter
- 18, 68: Öffnungseinrichtung
- 20, 70: Hülse
- 21: Schulter
- 22, 72: Verschlusskappe
- 23, 73: Öffnung
- 24, 74: Innengewinde
- 26, 76: Sicherungselement
- 27: Ampullenboden
- 28, 78: Griff
- 30, 80: Dichtung
- 32, 82: Dichtung
- 34, 84: Dorn
- 36, 86: Durchgang
- 37, 87: Stutzen
- 38, 88: Verschluss
- 39, 89: Flügel
- 40, 90: Innengewinde
- 42, 92: Außengewinde
- 43, 93: Dichtung
- 44: Schraubring
- 45: Außengewinde
- 46,96: Innengewinde
- 47: Leiste
- 48: Knochenzement
- 49: Austragsrohr
- 58: Nut
- 94: Verschiebering
- 95: Anschlag

## Patentansprüche

1. Knochenzementapplikator zum Lagern und Mischen eines Knochenzementpulvers (4) und einer Monomerflüssigkeit (3) sowie zum Applizieren eines aus dem Knochenzementpulver (4) und der Monomerflüssigkeit (3) gemischten pastösen Knochenzements (48), der Knochenzementapplikator aufweisend eine Kartusche (1, 51) mit einem zylindrischen Innenraum (11, 61) zum Mischen des Knochenzements (48), wobei die Kartusche (1, 51) an einer Vorderseite einen Kartuschenkopf (6, 56) mit einer Austragsöffnung zum Austreiben des Knochenzements (48) aus dem Innenraum (11, 61) aufweist,
einen Austragskolben (12, 62) zum Austreiben des gemischten Knochenzements (48) aus dem Innenraum (11, 61) durch die Austragsöffnung, wobei der Austragskolben (12, 62) im Innenraum (11, 61) der Kartusche (1, 51) entlang der Zylinderachse des Innenraums (11, 61) in Richtung des Kartuschenkopfs (6, 56) beweglich angeordnet ist, wobei das Knochenzementpulver (4) zwischen dem Austragskolben (12, 62) und dem Kartuschenkopf (6, 56) in dem Innenraum (11, 61) der Kartusche (1, 51) enthalten ist, eine Aufnahme (2, 52), wobei ein Monomerflüssigkeitsbehälter (5) im Inneren der Aufnahme (2, 52) angeordnet ist, wobei der Monomerflüssigkeitsbehälter (5) die Monomerflüssigkeit (3) enthält und im Inneren der Aufnahme (2, 52) zu öffnen ist, wobei die Aufnahme (2, 52) an einer der Vorderseite der Kartusche (1, 51) gegenüberliegenden Rückseite der Kartusche (1, 51) in der Kartusche (1, 51) steckt und in der Kartusche (1, 51) beweglich ist, und
einen Mischstab (7, 57), wobei der Mischstab (7, 57) mit einem daran befestigten Mischer (10, 60) in dem Innenraum (11, 61) der Kartusche (1, 51) angeordnet ist, wobei der Mischer (10, 60) an einer dem Kartuschenkopf (6, 56) zugewandten Vorderseite des Mischstabs (7, 57) befestigt ist, wobei der Mischstab (7, 57) an einer dem Mischer (10, 60) gegenüberliegenden Seite mit einer dem Kartuschenkopf (6, 56) zugewandten Vorderseite der Aufnahme (2, 52) verbunden ist, so dass der Mischstab (7, 57) mit dem Mischer (10, 60) in dem Innenraum (11, 61) zum Durchmischen des Knochenzementpulvers (4) mit der Monomerflüssigkeit (3) durch eine Bewegung der Aufnahme (2, 52) gegen die Kartusche (1, 51) beweglich ist, und wobei der Mischstab (7, 57) lösbar mit der Aufnahme (2, 52) verbunden ist und der von der Aufnahme (2, 52) gelöste Mischstab (7, 57) bei einem Vortreiben der Aufnahme (2, 52) in Richtung des Kartuschenkopfs (6, 56) in die Aufnahme (2, 52) hinein drückbar ist.

2. Knochenzementapplikator nach Anspruch 1, **dadurch gekennzeichnet, dass**
der Mischstab (7, 57) durch einen Druck auf den am Kartuschenkopf (6, 56) anliegenden Mischer (10, 60) und/oder durch ein Drehen oder Schrauben der Aufnahme (2, 52) gegen den gegen eine Drehung im Innenraum (11, 61) gesicherten Mischer (10, 60) von der Aufnahme (2, 52) lösbar ist.

3. Knochenzementapplikator nach einem der Ansprüche 1 oder 2, **dadurch**
**gekennzeichnet, dass**
die Vorderseite der Aufnahme (2, 52) den Austragskolben (12, 62) zum Austreiben des Knochenzements (48) aus dem Innenraum (11, 61) bildet, wobei bevorzugt der Austragskolben (12, 62) zylindrisch ist.

4. Knochenzementapplikator nach Anspruch 1 oder 2, **dadurch gekennzeichnet, dass** der Innenraum (11, 61) mit dem Austragskolben (12, 62) in einen vorderen Teil und einen hinteren Teil getrennt ist, wobei der vordere Teil des Innenraums (11, 61) durch den Kartuschenkopf (6, 56) und den Austragskolben (12, 62) begrenzt ist und der hintere Teil des Innenraums (11, 61) durch den Austragskolben (12, 62) und die Aufnahme (2, 52) begrenzt ist, wobei der Mischstab (7, 57) durch eine Durchführung in dem Austragskolben (12, 62) geführt ist und in der Durchführung axial beweglich gelagert ist, wobei der Mischer (10, 60) und das Knochenzementpulver (4) im vorderen Teil des Innenraums (11, 61) angeordnet sind und wobei der Austragskolben (12, 62) mit der Aufnahme (2, 52) in Richtung des Kartuschenkopfs (6, 56) drückbar ist.

5. Knochenzementapplikator nach einem der vorangehenden Ansprüche, **dadurch**
**gekennzeichnet, dass**
der Austragskolben (12, 62) zumindest einen für das Knochenzementpulver (4) undurchlässigen und für die Monomerflüssigkeit (3) und Gase durchlässigen Kanal (14, 64) hat.

6. Knochenzementapplikator nach einem der vorangehenden Ansprüche, **dadurch**
**gekennzeichnet, dass**
die Aufnahme (2, 52) bis zu einem durch ein Außengewinde (9, 59) an der Aufnahme (2, 52) gebildeten ersten Anschlag in den Innenraum (11, 61) hineinschiebbar ist und bis zu einem zweiten Anschlag mit dem Außengewinde (9, 59) in ein Innengewinde in der Kartusche (1, 51) an deren Rückseite oder in ein Innengewinde (46, 96) in einem Ring (44, 94) an der Rückseite der Kartusche (1, 51) einschraubbar ist, wobei der Mischstab (7, 57) bei einer Bewegung der Aufnahme (2, 52) bis zum ersten Anschlag nicht von der Aufnahme (2, 52) lösbar ist und der Mischstab (7, 57) durch Einschrauben der Aufnahme (2, 52) in die Kartusche (1, 51) von der Aufnahme (2, 52) lösbar ist.

7. Knochenzementapplikator nach einem der vorangehenden Ansprüche, **dadurch**
**gekennzeichnet, dass**
an der Aufnahme (2, 52) eine von außen bedienbare Öffnungseinrichtung (18, 68) angeordnet ist, mit der der Monomerflüssigkeitsbehälter (5) im Inneren der Aufnahme (2, 52) zu öffnen ist.

8. Knochenzementapplikator nach einem der vorangehenden Ansprüche, **dadurch**
**gekennzeichnet, dass**
das Innere der Aufnahme (2, 52) mit dem Innenraum (11, 61) der Kartusche (1, 51) flüssigkeitsdurchlässig verbunden ist, wobei bevorzugt hierzu die dem Kartuschenkopf (6, 56) zugewandte Vorderseite der Aufnahme (2, 52) wenigstens einen flüssigkeitsdurchlässigen Durchgang (36, 86) und der Austragskolben (12, 62) wenigstens einen flüssigkeitsdurchlässigen Kanal (14, 64) aufweisen.

9. Knochenzementapplikator nach einem der vorangehenden Ansprüche, **dadurch**
**gekennzeichnet, dass**
der Kartuschenkopf (6, 56) ein auf die Kartusche (1, 51) aufschraubbarer Kartuschendeckel (6, 56) ist, wobei der Kartuschendeckel (6, 56) den Innenraum (11, 61) der Kartusche (1, 51) an deren Vorderseite gasdicht und flüssigkeitsdicht abdichtet und wobei die Austragsöffnung in dem Kartuschendeckel (6, 56) angeordnet ist, vorzugsweise in einem Stutzen (37, 87) im Kartuschendeckel (6, 56) angeordnet ist.

10. Knochenzementapplikator nach einem der vorangehenden Ansprüche, **dadurch**
**gekennzeichnet, dass**
die Kartusche (1, 51) an deren Rückseite ein Innengewinde (8) und/oder einen Ring (44, 94) mit einem Innengewinde (46, 96) zum Einschrauben der Aufnahme (2, 52) aufweist, wobei an der Aufnahme (2, 52) ein zu dem Innengewinde (8) der Kartusche (1, 51) oder zu dem Innengewinde (46, 96) des Rings (44, 94) passendes Außengewinde (9, 59) vorgesehen ist.

11. Knochenzementapplikator nach einem der vorangehenden Ansprüche, **dadurch**
**gekennzeichnet, dass**
an der ins Innere der Aufnahme (2, 52) weisenden Seite der Aufnahme (2, 52) ein Dorn (34, 84) zum Öffnen des Monomerflüssigkeitsbehälters (5) angeordnet ist.

12. Knochenzementapplikator nach einem der vorangehenden Ansprüche, **dadurch**
**gekennzeichnet, dass**
der Mischstab (7, 57) in seiner Verbindung zur Aufnahme (2, 52) eine Kreisscheibe mit einem Außengewinde aufweist, wobei die Kreisscheibe in ein passendes Innengewinde an der dem Kartuschenkopf (6, 56) zugewandten Vorderseite der Aufnahme (2, 52) geschraubt ist, wobei bevorzugt das Außengewinde der Kreisscheibe und das Innengewinde an der Vorderseite der Aufnahme (2, 52) Linksgewinde sind.

13. Knochenzementapplikator nach einem der vorangehenden Ansprüche, **dadurch**
**gekennzeichnet, dass**
die Aufnahme (2, 52) an ihrer dem Kartuschenkopf (6, 56) gegenüberliegenden Rückseite einen größeren Durchmesser als der Innenraum (11, 61) der Kartusche (1, 51) aufweist.

14. Knochenzementapplikator nach einem der vorangehenden Ansprüche, **dadurch**
**gekennzeichnet, dass**
an der Rückseite der Kartusche (1, 51) ein Ring (44, 94) mit einem Gewinde (46, 96), bevorzugt mit einem Innengewinde (46, 96), angeordnet ist, wobei die Aufnahme (2, 52) ein zum Gewinde (46, 96) des Rings (44, 94) passendes Gegengewinde (9, 59), insbesondere ein passendes Außengewinde (9, 59), aufweist und wobei der Ring (44, 94) durch Verschieben oder durch Schrauben in axialer Richtung bezüglich der Zylinderachse des zylindrischen Innenraums (11, 61) der Kartusche (1, 51) gegen die Kartusche (1, 51) beweglich mit der Kartusche (1, 51) verbunden ist.

15. Verfahren zur Herstellung eines Knochenzements (48) mit einem Knochenzementapplikator nach einem der vorangehenden Ansprüche umfassend die folgenden Schritte
A) Öffnen des Monomerflüssigkeitsbehälters (5) im Inneren der Aufnahme (2, 52) und Ausfließen der Monomerflüssigkeit (3) aus dem Monomerflüssigkeitsbehälter (5), wobei die Monomerflüssigkeit (3) aus der Aufnahme (2, 52) in das Knochenzementpulver (4) im Innenraum (11, 61) der Kartusche (1, 51) fließt,
B) abwechselndes Herausziehen der Aufnahme (2, 52) aus dem Innenraum (11, 61) der Kartusche (1, 51) und Einschieben der Aufnahme (2, 52) in den Innenraum (11, 61) der Kartusche (1, 51), wobei durch diese Bewegung der Mischer (10, 60) in dem Innenraum (11, 61) der Kartusche (1, 51) bewegt wird und dadurch das Knochenzementpulver (4) und die Monomerflüssigkeit (3) miteinander zum Knochenzement (48) gemischt werden,
C) Lösen des Mischstabs (7, 57) von der Aufnahme (2, 52) durch eine Schraubbewegung oder Drehbewegung der Aufnahme (2, 52) gegen die Kartusche (1, 51) und/oder durch Einpressen der Aufnahme (2, 52) in den Innenraum (11, 61) der Kartusche (1, 51),
D) Öffnen der Austragsöffnung, und
E) Auspressen des Knochenzements (48) aus dem Innenraum (11, 61) der Kartusche (1, 51) durch die geöffnete Austragsöffnung, wobei der Knochenzement (48) mit dem Austragskolben (12, 62) aus dem Innenraum (11, 61) der Kartusche (1, 51) gepresst wird und der Austragskolben (12, 62) durch Eindrücken oder Einschrauben der Aufnahme (2, 52) in den Innenraum (11, 61) der Kartusche (1, 51) angetrieben wird und wobei der Mischstab (7, 57) in die Aufnahme (2, 52) hineingedrückt wird.

16. Verfahren nach Anspruch 15, **dadurch gekennzeichnet, dass**
der Austragskolben (12, 62) als separates Teil im Innenraum (11, 61) der Kartusche (1, 51) angeordnet ist und der Austragskolben (12, 62) wenigstens einen für das Knochenzementpulver (4) undurchlässigen und für die Monomerflüssigkeit (3) und Gase durchlässigen Kanal (14, 64) aufweist, wobei
in Schritt A) die Monomerflüssigkeit (3) durch den Austragskolben (12, 62) hindurch in den durch den Austragskolben (12, 62) und den Kartuschenkopf (6, 56) begrenzten vorderen Teil des Innenraums (11, 61) der Kartusche (1, 51)fließt, in Schritt B) der Mischstab (7, 57) durch eine Durchführung in dem Austragskolben (12, 62) bewegt wird und in Schritt E) der Austragskolben (12, 62) von der Aufnahme (2, 52) in Richtung des Kartuschenkopfs (6, 56) gedrückt wird.

## Claims

1. Bone cement applicator for storage and mixing of a bone cement powder (4) and a monomer liquid (3) as well as for application of a pasty bone cement (48) produced by mixing the bone cement powder (4) and the monomer liquid (3), the bone cement applicator comprising
a cartridge (1, 51) with a cylindrical internal space (11, 61) for mixing the bone cement (48), whereby the cartridge (1, 51) comprises, on a front side, a cartridge head (6, 56) with a dispensing opening for expelling the bone cement (48) from the internal space (11, 61);
a dispensing plunger (12, 62) for expelling the mixed bone cement (48) from the internal space (11, 61) through the dispensing opening, whereby the dispensing plunger (12, 62) is arranged such as to be mobile in the internal space (11, 61) of the cartridge (1, 51) along the cylinder axis of the internal space (11, 61) in the direction of the cartridge head (6, 56), whereby the bone cement powder (4) is contained between the dispensing plunger (12, 62) and the cartridge head (6, 56) in the internal space (11, 61) of the cartridge (1, 51); a receptacle (2, 52), whereby a monomer liquid container (5) is arranged on the inside of the receptacle (2, 52), whereby the monomer liquid container (5) contains the monomer liquid (3) and can be opened on the inside of the receptacle (2, 52), whereby the receptacle (2, 52) is plugged into the cartridge (1, 51) on a rear side of the cartridge (1, 51) that is situated opposite from the front side of the cartridge (1, 51), and is mobile in the cartridge (1, 51); and a mixing rod (7, 57), whereby the mixing rod (7, 57) with a mixer (10, 60) affixed to it is arranged in the internal space (11, 61) of the cartridge (1, 51), whereby the mixer (10, 60) is affixed on a front side of the mixing rod (7, 57) that faces the cartridge head (6, 56), whereby the mixing rod (7, 57) is connected, on a side that is situated opposite from the mixer (10, 60), to a front side of the receptacle (2, 52) that faces the cartridge head (6, 56), such that the mixing rod (7, 57) with the mixer (10, 60) is mobile in the internal space (11, 61) for the mixing of the bone cement powder (4) with the monomer liquid (3) through a motion of the receptacle (2, 52) against the cartridge (1, 51), and whereby the mixing rod (7, 57) is detachably connected to the receptacle (2, 52), and the mixing rod (7, 57), detached from the receptacle (2, 52), can be pushed into the receptacle (2, 52) upon the receptacle (2, 52) being propelled in the direction of the cartridge head (6, 56).

2. Bone cement applicator according to claim 1, **characterised in that** the mixing rod (7, 57) is detachable from the receptacle (2, 52) by pressing onto the mixer (10, 60) touching against the cartridge head (6, 56), and/or by rotating or screwing the receptacle (2, 52) against the mixer (10, 60), which is secured against a rotation in the internal space (11, 61).

3. Bone cement applicator according to claim 1 or 2, **characterised in that** the front side of the receptacle (2, 52) forms the dispensing plunger (12, 62) for expelling the bone cement (48) from the internal space (11, 61), whereby the dispensing plunger (12, 62) is preferred to be cylindrical.

4. Bone cement applicator according to claim 1 and 2, **characterised in that** the internal space (11, 61) with the dispensing plunger (12, 62) is separated into a front part and a rear part, whereby the front part of the internal space (11, 61) is bordered by the cartridge head (6, 56) and the dispensing plunger (12, 62) and the rear part of the internal space (11, 61) is bordered by the dispensing plunger (12, 62) and the receptacle (2, 52), whereby the mixing rod (7, 57) is conducted through a feedthrough in the dispensing plunger (12, 62) and is supported in the feedthrough such as to be axially mobile, whereby the mixer (10, 60) and the bone cement powder (4) are arranged in the front part of the internal space (11, 61), and whereby the dispensing plunger (12, 62) can be pushed in the direction of the cartridge head (6, 56) by the receptacle (2, 52).

5. Bone cement applicator according to any one of the preceding claims,
**characterised in that**
the dispensing plunger (12, 62) comprises at least one channel (14, 64) that is impermeable to the bone cement powder (4) and is permeable to the monomer liquid (3) and gases.

6. Bone cement applicator according to any one of the preceding claims,
**characterised in that**
the receptacle (2, 52) can be inserted into the internal space (11, 61) up to a first limit stop that is formed by an external thread (9, 59) on the receptacle (2, 52) and can be screwed in up to a second limit stop, by means of the external thread (9, 59), into an internal thread in the cartridge (1, 51) on the rear side thereof or into an internal thread (46, 96) in a ring (44, 94) on the rear side of the cartridge (1, 51), whereby the mixing rod (7, 57) cannot be detached from the receptacle (2, 52) upon a motion of the receptacle (2, 52) up to the first limit stop, and the mixing rod (7, 57) can be detached from the receptacle (2, 52) by screwing the receptacle (2, 52) into the cartridge (1, 51).

7. Bone cement applicator according to any one of the preceding claims,
**characterised in that**
an opening facility (18, 68), which can be operated from outside and by means of which the monomer liquid container (5) can be opened on the inside of the receptacle (2, 52), is arranged on the receptacle (2, 52).

8. Bone cement applicator according to any one of the preceding claims,
**characterised in that**
the inside of the receptacle (2, 52) is connected in liquid-permeable manner to the internal space (11, 61) of the cartridge (1, 51), whereby, for this purpose, the front side of the receptacle (2, 52) facing the cartridge head (6, 56) preferably comprises at least one liquid-permeable passage (36, 86), and the dispensing plunger (12, 62) comprises at least one liquid-permeable channel (14, 64).

9. Bone cement applicator according to any one of the preceding claims,
**characterised in that**
the cartridge head (6, 56) is a cartridge lid (6, 56) that can be screwed onto the cartridge (1, 51), whereby the cartridge lid (6, 56) seals the internal space (11, 61) of the cartridge (1, 51) on the front side thereof in gas-tight and liquid-tight manner, and whereby the dispensing opening is arranged in the cartridge lid (6, 56), preferably is arranged in a socket (37, 87) in the cartridge lid (6, 56).

10. Bone cement applicator according to any one of the preceding claims,
**characterised in that**
the cartridge (1, 51) comprises, on the rear side thereof, an internal thread (8) and/or a ring (44, 94) with an internal thread (46, 96) for screwing-in the receptacle (2, 52), whereby an external thread (9, 59) fitting the internal thread (8) of the cartridge (1, 51) or the internal thread (46, 96) of the ring (44, 94) is provided on the receptacle (2, 52).

11. Bone cement applicator according to any one of the preceding claims,
**characterised in that**
a mandrel (34, 84) for opening of the monomer liquid container (5) is arranged on the side of the receptacle (2, 52) pointing into the inside of the receptacle (2, 52).

12. Bone cement applicator according to any one of the preceding claims,
**characterised in that**
the mixing rod (7, 57) comprises, in its connection to the receptacle (2, 52), a circular disk with an external thread, whereby the circular disk is screwed into a fitting internal thread on the front side of the receptacle (2, 52) facing the cartridge head (6, 56), whereby the external thread of the circular disk and the internal thread on the front side of the receptacle (2, 52) are preferred to be left-hand threads.

13. Bone cement applicator according to any one of the preceding claims,
**characterised in that**
the receptacle (2, 52), on its rear side situated opposite from the cartridge head (6, 56), has a larger diameter than the internal space (11, 61) of the cartridge (1, 51).

14. Bone cement applicator according to any one of the preceding claims,
**characterised in that**
a ring (44, 94) with a thread (46, 96), preferably with an internal thread (46, 96), is arranged on the rear side of the cartridge (1, 51), whereby the receptacle (2, 52) comprises a counter-thread (9, 59) that fits the thread (46, 96) of the ring (44, 94), in particular a fitting external thread (9, 59), and whereby the ring (44, 94) is connected in mobile manner to the cartridge (1, 51) by shifting or by screwing in axial direction with respect to the cylinder axis of the cylindrical internal space (11, 61) of the cartridge (1, 51) against the cartridge (1, 51).

15. Method for producing a bone cement (48) with a bone cement applicator according to any one of the preceding claims, comprising the following steps of
A) Opening the monomer liquid container (5) on the inside of the receptacle (2, 52) and the monomer liquid (3) flowing from the monomer liquid container (5), whereby the monomer liquid (3) flows from the receptacle (2, 52) into the bone cement powder (4) in the internal space (11, 61) of the cartridge (1, 51);
B) alternatingly pulling the receptacle (2, 52) from the internal space (11, 61) of the cartridge (1, 51) and inserting the receptacle (2, 52) into the internal space (11, 61) of the cartridge (1, 51), whereby said motion moves the mixer (10, 60) in the internal space (11, 61) of the cartridge (1, 51) and thus mixes the bone cement powder (4) and the monomer liquid (3) with each other to form bone cement (48);
C) detaching the mixing rod (7, 57) from the receptacle (2, 52) through a screwing motion or rotating motion of the receptacle (2, 52) against the cartridge (1, 51) and/or by pressing the receptacle (2, 52) into the internal space (11, 61) of the cartridge (1, 51);
D) opening the dispensing opening; and
E) extruding the bone cement (48) from the internal space (11, 61) of the cartridge (1, 51) through the opened dispensing opening, whereby the bone cement (48) is extruded from the internal space (11, 61) of the cartridge (1, 51) by means of the dispensing plunger (12, 62), and the dispensing plunger (12, 62) is driven by pushing or screwing the receptacle (2, 52) into the internal space (11, 61) of the cartridge (1, 51)), and whereby the mixing rod (7, 57) is being pushed into the receptacle (2, 52).

16. Method according to claim 15, **characterised in that**
the dispensing plunger (12, 62) is arranged as a separate part in the internal space (11, 61) of the cartridge (1, 51), and the dispensing plunger (12, 62) comprises at least one channel (14, 64) that is impermeable to the bone cement powder (4) and is permeable to the monomer liquid (3) and gases, whereby
in step A), the monomer liquid (3) flows through the dispensing plunger (12, 62) into the front part of the internal space (11, 61) of the cartridge (1, 51) that is bordered by the dispensing plunger (12, 62) and the cartridge head (6, 56), in step B), the mixing rod (7, 57) is being moved through a feedthrough in the dispensing plunger (12, 62),
and in step E), the dispensing plunger (12, 62) is being pushed in the direction of the cartridge head (6, 56) by the receptacle (2, 52).

## Revendications

1. Applicateur de ciment osseux pour le stockage et le mélange d'une poudre de ciment osseux (4) et d'un liquide monomère (3) ainsi que pour l'application d'un ciment osseux (48) pâteux, mélangé à partir de la poudre de ciment osseux (4) et du liquide monomère (3), l'applicateur de ciment osseux présentant
une cartouche (1, 51) avec un espace intérieur (11, 61) cylindrique pour le mélange du ciment osseux (48), la cartouche (1, 51) présentant, sur une face avant, une tête de cartouche (6, 56) avec un orifice de sortie pour l'éjection du ciment osseux (48) de l'espace intérieur (11, 61),
un piston de décharge (12, 62) pour l'éjection du ciment osseux (48) mélangé de l'espace intérieur (11, 61) par l'orifice de sortie, le piston de décharge (12, 62) dans l'espace intérieur (11, 61) de la cartouche (1, 51) étant déplaçable le long de l'axe cylindrique de l'espace intérieur (11, 61) en direction de la tête de cartouche (6, 56), la poudre de ciment osseux (4) étant contenue entre le piston de décharge (12, 62) et la tête de cartouche (6, 56) dans l'espace intérieur (11, 61) de la cartouche (1, 51),
un logement (2, 52), un réservoir de liquide monomère (5) étant disposé à l'intérieur du logement (2, 52), le réservoir de liquide monomère (5) contenant le liquide monomère (3) et pouvant être ouvert à l'intérieur du logement (2, 52), le logement (2, 52) s'insérant dans la cartouche (1, 51) sur une face arrière de la cartouche (1, 51), opposée à la face avant de la cartouche (1, 51), et étant déplaçable dans la cartouche (1, 51) et
une barre de mélange (7, 57), la barre de mélange (7, 57) étant disposée avec un mélangeur (10, 60) fixé sur celle-ci dans l'espace intérieur (11, 61) de la cartouche (1, 51), le mélangeur (10, 60) étant fixé sur une face avant de la barre de mélange (7, 57), tournée vers la tête de cartouche (6, 56), la barre de mélange (7, 57) étant reliée, sur une face opposée au mélangeur (10, 60), à une face avant du logement (2, 52), tournée vers la tête de cartouche (6, 56), de façon à ce que la barre de mélange (7, 57) soit déplaçable avec le mélangeur (10, 60) dans l'espace intérieur (11, 61) pour le mélange de la poudre de ciment osseux (4) avec le liquide monomère (3) par un déplacement du logement (2, 52) contre la cartouche (1, 51), la barre de mélange (7, 57) étant reliée au logement (2, 52) de manière amovible et la barre de mélange (7, 57) retirée du logement (2, 52) pouvant être enfoncée dans le logement (2, 52) lors d'un avancement du logement (2, 52) en direction de la tête de cartouche (6, 56).

2. Applicateur de ciment osseux conformément à la revendication n°1,
**caractérisé en ce que**
la barre de mélange (7,57) peut être retirée du logement (2, 52) par une pression sur le mélangeur (10, 60) adjacent à la tête de cartouche (6, 56) et/ou par une rotation ou un vissage du logement (2, 52) contre le mélangeur (10, 60) sécurisé contre une rotation dans l'espace intérieur (11, 61).

3. Applicateur de ciment osseux conformément à l'une des revendications n°1 ou n°2, **caractérisé en ce que**
la face avant du logement (2, 52) constitue le piston de décharge (12, 62) pour l'éjection du ciment osseux (48) de l'espace intérieur (11, 61), le piston de décharge (12, 62) étant préférablement cylindrique.

4. Applicateur de ciment osseux conformément à la revendication n°1 ou n°2,
**caractérisé en ce que**
l'espace intérieur (11, 61) avec le piston de décharge (12, 62) est séparé dans une partie avant et une partie arrière, la partie avant de l'espace intérieur (11, 61) étant limitée par la tête de cartouche (6, 56) et le piston de décharge (12, 62) et la partie arrière de l'espace intérieur (11, 61) étant limitée par le piston de décharge (12, 62) et le logement (2, 52), la barre de mélange (7, 57) étant guidée par un passage dans le piston de décharge (12, 62) et déplaçable axialement dans le passage, le mélangeur (10, 60) et la poudre de ciment osseux (4) étant disposés dans la partie avant de l'espace intérieur (11, 61) et le piston de décharge (12, 62) pouvant être enfoncé avec le logement (2, 52) en direction de la tête de cartouche (6, 56).

5. Applicateur de ciment osseux conformément à l'une des revendications précédentes, **caractérisé en ce que**
le piston de décharge (12, 62) possède au moins un conduit (14, 64) imperméable à la poudre de ciment osseux (4) et perméable au liquide monomère (3) et aux gaz.

6. Applicateur de ciment osseux conformément à l'une des revendications précédentes, **caractérisé en ce que**
le logement (2, 52) peut être enfoncé dans l'espace intérieur (11, 61) jusqu'à une première butée formée par un filetage extérieur (9, 59) sur le logement (2, 52) et peut être vissé jusqu'à une deuxième butée avec le filetage extérieur (9, 59) dans un filetage intérieur dans la cartouche (1, 51) sur sa face arrière ou dans un filetage intérieur (46, 96) dans un anneau (44, 94) sur la face arrière de la cartouche (1, 51), la barre de mélange (7, 57) ne pouvant être retirée du logement (2, 52) lors d'un déplacement du logement (2, 52) jusqu'à la première butée et la barre de mélange (7, 57) pouvant être retirée du logement (2, 52) par le vissage du logement (2, 52) dans la cartouche (1, 51).

7. Applicateur de ciment osseux conformément à l'une des revendications précédentes, **caractérisé en ce que**
un dispositif d'ouverture (18, 68) pouvant être utilisé depuis l'extérieur, avec lequel le réservoir de liquide monomère (5) peut être ouvert à l'intérieur du logement (2, 52), est disposé sur le logement (2, 52).

8. Applicateur de ciment osseux conformément à l'une des revendications précédentes, **caractérisé en ce que**
l'intérieur du logement (2, 52) est relié, en étant perméable aux liquides, à l'espace intérieur (11, 51) de la cartouche (1, 51), la face avant du logement (2, 52), tournée vers la tête de cartouche (6, 56), présentant préférablement au moins un passage (36, 86) perméable aux liquides et le piston de décharge (12, 62) présentant au moins un conduit (14, 64) perméable aux liquides.

9. Applicateur de ciment osseux conformément à l'une des revendications précédentes, **caractérisé en ce que**
la tête de cartouche (6, 56) est un couvercle de cartouche (6, 56) dévissable sur la cartouche (1, 51), le couvercle de cartouche (6, 56) étanchéifiant hermétiquement aux gaz et aux liquides l'espace intérieur (11, 61) de la cartouche (1, 51) sur sa face avant et l'orifice de sortie étant disposé dans le couvercle de cartouche (6, 56), préférablement dans un embout (37, 87) dans le couvercle de cartouche (6, 56).

10. Applicateur de ciment osseux conformément à l'une des revendications précédentes, **caractérisé en ce que**
la cartouche (1, 51) présente, sur sa face arrière, un filetage intérieur (8) et/ou un anneau (44, 94) avec un filetage intérieur (46, 96) pour le vissage du logement (2, 52), un filetage extérieur (9, 59) adapté au filetage intérieur (8) de la cartouche (1, 51) ou au filetage intérieur (46, 96) de l'anneau (44, 94) étant prévu sur le logement (2, 52).

11. Applicateur de ciment osseux conformément à l'une des revendications précédentes, **caractérisé en ce que**
un mandrin (34, 84) pour l'ouverture du réservoir de liquide monomère (5) est disposé sur la face du logement (2, 52), tournée vers l'intérieur du logement (2, 52).

12. Applicateur de ciment osseux conformément à l'une des revendications précédentes, **caractérisé en ce que**
la barre de mélange (7, 57) présente, dans sa liaison avec le logement (2, 52), un disque avec un filetage extérieur, le disque étant vissé dans un filetage intérieur adéquat sur la face avant du logement (2, 52), tournée vers la tête de cartouche (6, 56), le filetage extérieur du disque et le filetage intérieur sur la face avant du logement (2, 52) étant préférablement des filetages à gauche.

13. Applicateur de ciment osseux conformément à l'une des revendications précédentes, **caractérisé en ce que**
le logement (2, 52) présente, sur sa face arrière opposée à la tête de cartouche (6, 56), un plus grand diamètre que l'espace intérieur (11, 61) de la cartouche (1, 51).

14. Applicateur de ciment osseux conformément à l'une des revendications précédentes, **caractérisé en ce que**
un anneau (44, 94) avec un filetage (46, 96), préférablement avec un filetage intérieur (46, 96), est disposé sur la face arrière de la cartouche (1, 51), le logement (2, 52) présentant un contre-filetage (9, 59) adapté au filetage (46, 96) de l'anneau (44, 94), en particulier un filetage extérieur (9, 59) adapté, et l'anneau (44, 94) étant relié de façon mobile à la cartouche (1, 51) par un déplacement ou un vissage dans le sens axial par rapport à l'axe cylindrique de l'espace intérieur (11, 61) cylindrique de la cartouche (1, 51) contre la cartouche (1, 51).

15. Procédé de fabrication d'un ciment osseux (48) avec un applicateur de ciment osseux conformément à l'une des revendications précédentes, comprenant les étapes suivantes :
A) Ouverture du réservoir de liquide monomère (5) à l'intérieur du logement (2, 52) et écoulement du liquide monomère (3) du réservoir de liquide monomère (5), le liquide monomère (3) s'écoulant du logement (2, 52) dans la poudre de ciment osseux (4) dans l'espace intérieur (11, 61) de la cartouche (1, 51)
B) En alternance, retrait du logement (2, 52) de l'espace intérieur (11, 61) de la cartouche (1, 51) et insertion du logement (2, 52) dans l'espace intérieur (11, 61) de la cartouche (1, 51), le mélangeur (10, 60) étant déplacé dans l'espace intérieur (11, 61) de la cartouche (1, 51) par ce déplacement et la poudre de ciment osseux (4) et le liquide monomère (3) étant ainsi mélangés en un ciment osseux (48)
C) Retrait de la barre de mélange (7, 57) du logement (2, 52) par un mouvement de vissage ou un mouvement de rotation du logement (2, 52) contre la cartouche (1, 51) et/ou par pression du logement (2, 52) dans l'espace intérieur (11, 61) de la cartouche (1, 51)
D) Ouverture de l'orifice de sortie et
E) Pressage du ciment osseux (48) de l'espace intérieur (11, 61) de la cartouche (1, 51) par l'orifice de sortie ouvert, le ciment osseux (48) étant pressé de l'espace intérieur (11, 61) de la cartouche (1, 51) avec le piston de décharge (12, 62) et le piston de décharge (12, 62) étant entraîné par la pression ou le vissage du logement (2, 52) dans l'espace intérieur (11, 61) de la cartouche (1, 51) et la barre de mélange (7, 57) étant enfoncée dans le logement (2, 52).

16. Procédé conformément à la revendication n°15, **caractérisé en ce que** le piston de décharge (12, 62) est disposé comme pièce séparée dans l'espace intérieur (11, 61) de la cartouche (1, 51) et le piston de décharge (12, 62) présente au moins un conduit (14, 64) imperméable à la poudre de ciment osseux (4) et perméable au liquide monomère (3) et aux gaz,
dans l'étape A), le liquide monomère (3) coulant dans la partie avant de l'espace intérieur (11, 61) de la cartouche (1, 51), limitée par le piston de décharge (12, 62) et la tête de cartouche (6, 56), en passant par le piston de décharge (12, 62), dans l'étape B), la barre de mélange (7, 57) étant déplacée par un passage dans le piston de décharge (12, 62) et, dans l'étape E), le piston de décharge (12, 62) étant enfoncé depuis le logement (2, 52) en direction de la tête de cartouche (6, 56).
